# EUROPEAN PATENT SPECIFICATION

(11) **EP 4 216 892 B1**
(45) Date of publication and mention of the grant of the patent: **19.06.2024**
(21) Application number: 22765686.5
(22) Date of filing: 17.08.2022
(51) Int. Cl.: A61F 13/15, B65G 47/24, B65H 39/14

(54) **ABSORBENT ARTICLE INSERT CUTTING AND TRANSFER APPARATUS WITH SERVO MOTOR SUB-SYSTEM**
SCHNEIDE- UND TRANSFERVORRICHTUNG FÜR EINSATZ EINES SAUGFÄHIGEN ARTIKELS MIT SERVOMOTORUNTERSYSTEM
APPAREIL DE COUPE ET DE TRANSFERT D'INSERT D'ARTICLE ABSORBANT AVEC SOUS-SYSTÈME DE SERVOMOTEUR

(30) Priority: 17.08.2021 US 202163260315 P
(43) Date of publication of application: 02.08.2023
(73) Proprietor: Curt G. Joa, Inc., Sheboygan Falls, WI 53085 (US)
(72) Inventor: HORNESS, Darren, R., Sheboygan Falls, WI 53085 (US); KREIF, Lloyd, F., Sheboygan Falls, WI 53085 (US); FRITZ, Jeffrey, W., Sheboygan Falls, WI 53085 (US); DAVENPORT, Daniel, S., Collegeville, PA 19426 (US); WEAVER, Philip, A., Glenmoore, PA 19343 (US); BRYANT, Jason, C., Erie, PA 16505 (US); BIBA, Scott, I., Waunakee, WI 53597 (US)
(74) Representative: Potter Clarkson
(86) International application number: PCT/US2022/040652
(87) International publication number: WO 2023/023191

(56) References cited:
- EP-A1- 1 820 757
- US-A1- 2003 079 330
- US-A1- 2016 339 595
- US-A1- 2019 241 393

## Description

### BACKGROUND OF THE INVENTION

Embodiments of the invention relate to an apparatus for receiving and cutting a continuous web and transferring discrete articles or inserts, such as absorbent pads cut from the web, in the manufacture of disposable absorbent articles such as diapers, incontinence control garments, or female sanitary pads as they advance along a production line. More particularly, embodiments of the invention relate to such an apparatus being operable to accommodate cutting and transferring of discrete articles falling within a large range of differing types and sizes, with the apparatus being configurable to provide for timely and efficient adjustments in operation thereof to accommodate a product size change. A servo motor sub-system is provided in the apparatus that provides an increased pitch range at which the articles or inserts may be spaced, to accommodate larger product sizes.

In the production and manufacture of disposable products such as sanitary napkins or pants-type diapers, it frequently becomes necessary to manufacture a component of the product in one orientation, and then to spin that component part to a predetermined angle, which is suitably oriented for use in another step in the production process. As an example, a typical article or web to be reoriented is an absorbent article. Existing apparatuses function to receive a continuous web, cut a section from the web to form a discrete article, spin the article to a predetermined angle, and transfer the article for placement on a receiving surface. Additionally, the apparatus may also function to control a velocity and pitch between cut articles to achieve a desired placement pitch on the receiving surface. In the case of a diaper, for example, the article may be an absorbent insert to be placed on a fluid impervious chassis. Therefore, the web may be cut at a cut pitch, while the articles are deposited onto a receiving chassis web at a receiving pitch that matches a distance between consecutive chassis to be formed, with the receiving pitch defined by a distance extending from a chassis trailing edge, over an interval space, and to a subsequent chassis leading edge.

With regard to the structure of the apparatus, the apparatus is generally constructed to include a transfer device and a cutting system. The transfer device includes a large wheel (a "puck wheel") having a plurality of rotating pucks secured thereto that are selectively operable to provide the rotating and re-pitching of the discrete articles. The puck wheel is driven and supported by a shaft extending from the drive side of the apparatus, with the pucks in turn being rotated along with the wheel. Additionally, each of the pucks functions to spin/turn about its own spin axis, so as to provide for turning (e.g., 90-degree turn) of the discrete articles. The cutting system includes an anvil wheel and a cutting roll that interact with each other to cut the continuous web once it is received on a puck of the transfer device. The anvil wheel includes a plurality of anvils arranged thereon so as to be interspersed with the pucks. The anvil wheel is driven and supported by a shaft that may be common to or separate from the shaft driving the puck wheel, with the anvils rotating about the anvil wheel to periodically come into contact with a blade on the cutting roll to thereby cut the continuous web and form a discrete article that is held by a respective puck of the transfer device.

While existing cutting and transfer apparatuses as described above perform adequately for rotating and re-pitching pads for placement on a receiving surface, it is recognized that these apparatuses lack the flexibility desired in many manufacturing settings and implementations. As an example, it is often desirable for a single apparatus to be used in the manufacturing of a plurality of different disposable product types and sizes, with such a "size change" in the product being manufactured requiring: reconfiguring of the apparatus to accommodate different size articles on the pucks, operation of the apparatus at a different speed/velocity profile, modifying a pitch range for the pucks, and changing the positioning of the anvil wheel relative to the puck wheel and/or the cutting roll, as examples. Accordingly, in order to implement a different process flow or accommodate a size change from a product currently being manufactured, it is necessary to manually reconfigure, reposition, or swap out numerous components in the apparatus or replace the apparatus altogether with an apparatus capable of accommodating the dimensions of the discrete article and pitch range necessitated by the size change. Performing such modifications to the cutting and transfer apparatus is a difficult and time-consuming process that may require expert operator knowledge and increase downtime of the apparatus. Additionally, existing cutting and transfer apparatuses may be unable to accommodate products of a very large size due to limitations regarding achievable pitch range and puck velocity and movement, for example.

Therefore, it is desirable to provide a transfer device and overall cutting and transfer apparatus that is easily configurable, so as to accommodate the transferring and cutting of discrete articles of differing types and sizes. The device and apparatus would ensure that such reconfiguring is achieved while maintaining proper operation and arrangement of the various system components, to ensure proper interaction between such components. The device and apparatus would also be able to accommodate products of larger sizes.
US 2019/241393 A1 discloses a web cutting system for use with a single transfer insert placement mechanism having at least one puck for transferring a discrete web and a continuous web feeding mechanism for feeding a continuous web wherein first and second rollers having substantially parallel axes and being aligned with one another form a nip at their juncture, an anvil is attached to one roller, a die is attached to the other roller, a vacuum source is coupled to one of the rollers, a plurality of vacuum apertures is formed in the same roller. One of the rollers is positioned adjacent to the single transfer insert placement mechanism and to the continuous web feeding mechanism. The continuous web is applied to one roller and at least one discrete web is transferred from the puck to the same roller after which a die cutting process of the web occurs at the nip.
US 2003/0079330 A1 discloses a method and apparatus for changing the speed of discrete components can include a first rotatable drive member, and at least a second rotatable drive member which is substantially coaxial with the first drive member. A first servo motor is connected to rotate the first drive member, and at least a second servo motor is connected to rotate the second drive member. A first transfer puck is driven by the first rotatable drive member, and at least a second transfer puck is driven by the second rotatable drive member. A first electronic drive is connected to the first servo motor, and is configured to selectively move the first transfer puck at a first, pick up speed and at least a second, deposit speed. At least a second electronic drive is connected to the second servo motor, and is configured to selectively move the second transfer puck at the first pick up speed and at the second deposit speed.

### BRIEF DESCRIPTION OF THE INVENTION

In accordance with one aspect of the invention, a cutting and transfer apparatus includes a cutting system configured to cut an incoming continuous web of material into a plurality of discrete articles and a transfer mechanism operable with the cutting system to transfer and rotate the plurality of discrete articles from at least a web receiving location to an article placement location. The transfer mechanism further includes a drive shaft rotatable about a transfer axis and a puck wheel mounted to the drive shaft to rotate therewith about the transfer axis. The puck wheel includes a plurality of carriage units that rotate about the transfer axis to travel along a transfer path about the transfer axis from at least the web receiving location to the article placement location, each of the plurality of carriage units including a puck that is selectively operable to carry a discrete article thereon and reorient the article as the puck travels between the web receiving location and the article placement location. The puck wheel also includes a plurality of servo motors each operably connected to a respective carriage unit of the plurality of carriage units via a connecting arm, and with each of the plurality of servo motors operable to alter positioning of its respective carriage unit along at least a portion of the transfer path.

In accordance with another aspect of the invention, a pick-and-place system for transferring and rotating a plurality of discrete articles from at least an article receiving location to an article placement location is disclosed. The pick-and-place system includes a drive shaft rotatable about a transfer axis, a center plate mounted to the drive shaft to rotate therewith about the transfer axis, and a plurality of carriage units positioned about the center plate to rotate therewith to travel along a transfer path about the transfer axis from at least the article receiving location to the article placement location, with each of the plurality of carriage units including a puck that is selectively operable to carry a discrete article thereon and reorient the article as the puck travels between the article receiving location and the article placement location. The pick-and-place system also includes a plurality of servo motors mounted to the center plate, with each servo motor operably connected to a respective carriage unit of the plurality of carriage units via a connecting arm, and with each of the plurality of servo motors operable to alter positioning of its respective carriage unit with respect to the center plate along at least a portion of the transfer path.

In accordance with an embodiment that is not in accordance with the claims, a cutting and transfer apparatus includes a cutting system configured to cut an incoming web of material into a plurality of discrete articles and a transfer mechanism operable with the cutting system to transfer and rotate the plurality of discrete articles from at least a web receiving location to an article placement location. The transfer mechanism further includes a drive shaft rotatable about a transfer axis, a center plate mounted to the drive shaft to rotate therewith about the transfer axis, and a plurality of carriage units positioned about the center plate to rotate therewith to travel along a transfer path about the transfer axis from at least the web receiving location to the article placement location, with each of the plurality of carriage units including a puck that is selectively operable to carry a discrete article thereon and reorient the article as the puck travels between the web receiving location and the article placement location. The cutting system includes an anvil wheel having an anvil hub coupled to and driven by an anvil shaft to rotate about an anvil wheel axis and a plurality of anvil arms pivotably connected to and extending radially outward from the anvil hub, each of the plurality of anvil arms having thereon an anvil that cooperates with a knife roll of the cutting system to cut the incoming web of material. The plurality of anvil arms is equal in number to the plurality of carriage units, with the plurality of anvil arms interspersed with the plurality of carriage units such that each anvil arm is positioned between a pair of adjacent carriage units. Each anvil arm is mechanically coupled to the pair of adjacent carriage units between which it is positioned to control positioning of the anvil relative to the pair of adjacent carriage units.

In accordance with still another aspect of the invention, a method for configuring a cutting and transfer apparatus includes providing a cutter mechanism configured to cut an incoming web of material into a plurality of discrete articles and providing a transfer mechanism operable with the cutter mechanism to transfer and rotate the plurality of discrete articles from at least a web receiving location to an article placement location. In providing the transfer mechanism, the method further includes providing a drive shaft having a mounting structure coupled thereto, the drive shaft and mounting structure rotatable about a transfer axis and mounting a plurality of carriage units to the mounting structure so that the plurality of carriage units are rotatable with the mounting structure to travel along a transfer path about the transfer axis from at least the web receiving location to the pad placement location, with each of the plurality of carriage units including a puck that is selectively operable to carry a discrete article thereon and reorient the article as the puck travels between the web receiving location and the article placement location. The method also includes operably connecting a servo motor to each of the plurality of carriage units via a connecting arm, with each servo motor selectively operable to alter positioning of its respective carriage unit with respect to the mounting structure by circumferentially displacing the carriage unit along the transfer path.

In accordance with still another embodiment that is not in accordance with the claims, a cutting and transfer apparatus includes a cutting system configured to cut an incoming web of material into a plurality of discrete articles and a transfer mechanism operable with the cutting system to transfer and rotate the plurality of discrete articles from at least a web receiving location to an article placement location. The transfer mechanism further includes a drive shaft rotatable about a transfer axis, a mounting structure rotatable about the transfer axis, a plurality of carriage units coupled to the mounting structure and configured to travel along a transfer path about the transfer axis from at least the web receiving location to the article placement location, and a plurality of pucks coupled to the plurality of carriage units to carry discrete articles thereon and reorient the articles as the plurality of pucks travel between the web receiving location and the article placement location. The cutting system includes an anvil wheel having an anvil hub coupled to and driven by an anvil shaft to rotate about an anvil wheel axis and a plurality of anvil arms pivotably connected to and extending radially outward from the anvil hub, each of the plurality of anvil arms having thereon an anvil that cooperates with a knife roll of the cutting system to cut the incoming web of material. The plurality of anvil arms is equal in number to the plurality of carriage units, with the plurality of anvil arms interspersed with the plurality of carriage units such that each anvil arm is positioned between a pair of adjacent carriage units. Each anvil arm is mechanically coupled to the pair of adjacent carriage units between which it is positioned to control positioning of the anvil relative to the pair of adjacent carriage units.

In accordance with other aspects of the invention, such other aspects further include:
Wherein the puck wheel comprises a center plate mounted to the drive shaft to rotate therewith about the transfer axis, with the plurality of carriage units mounted to and positioned about the center plate and with the plurality of servo motors mounted to the center plate; and wherein each of the plurality of servo motors is operable to alter positioning of its respective carriage unit with respect to the center plate along at least the portion of the transfer path.

An annular rail structure positioned about an outer circumference of the center plate, the rail structure including pitch rails on which the plurality of carriage units is mounted to be movable therealong.

Wherein each of the plurality of carriage units comprises a chassis including a pair of frame members positioned on opposing sides of the rail structure; a plurality of rollers coupled to the chassis and in rolling engagement with an inner edge and outer edge of the pitch rails to secure the carriage unit to the rail structure and provide for translation of the carriage unit along the pitch rails; and a puck support positioned on the chassis and movable relative thereto via a mating of rail guides of the puck support with the pitch rails, the puck support oriented generally orthogonal to the pitch rails and comprising a puck mount on one end thereof that is configured to receive the puck.

Wherein the carriage unit comprises a drag link that couples the connecting arm to the chassis, with the drag link rotatably coupled to each of the connecting arm and to a mount on one of the frame members of the pair of frame members; and wherein the drag link transforms rotational motion of the servo motor and the connecting arm to a translation of the carriage unit along the pitch rails.

Wherein the transfer mechanism comprises a barrel cam stationarily situated about the transfer axis, the barrel cam having a spin cam race formed around an outer circumference thereof; and wherein each of the plurality of carriage units comprises a spin cam follower positioned on another end of the puck support, the spin cam follower in sliding or rolling communication with the spin cam race to spin the puck at least partially about a spin axis of the respective puck that is at least substantially perpendicular to the transfer axis, so as to reorient the article carried on the puck.

Wherein the transfer mechanism comprises a stationary vacuum manifold positioned adjacent the center plate and configured to transfer a vacuum from a vacuum source to an interior volume of the center plate; and a plurality of vacuum tubes coupled to the center plate, with one or more vacuum tubes of the plurality of vacuum tubes fluidly connecting the interior volume of the center plate with a respective carriage unit of the plurality of carriage units to transfer the vacuum thereto.

Wherein the plurality of vacuum tubes comprise telescoping vacuum tubes each rotatably coupled to the center plate to swivel relative thereto, and wherein each telescoping vacuum tube is collapsible and extendible to decrease and increase a length thereof, such that a length of the telescoping vacuum tube can change to accommodate movement of a respective carriage unit along the rail structure.

Wherein the puck support comprises one or more vacuum channels formed therein to provide a fluid flow path from one or more respective vacuum tubes to the puck mount and the puck mounted thereon.

Wherein the cutting system comprises a knife roll including one or more knives thereon; and an anvil wheel comprising an anvil hub coupled to and driven by an anvil shaft to rotate about an anvil wheel axis; a plurality of anvil arms pivotably connected to and extending radially outward from the anvil hub; and an anvil positioned at an end of each of the plurality of anvil arms, with the anvils periodically cooperating with the one or more knives of the knife roll to cut the incoming web of material; and a linkage system that mechanically couples the plurality of anvil arms together.

Wherein the linkage system comprises a pair of scissor links provided between each adjacent pair of anvil arms of the plurality of anvil arms, with a first link of the pair of scissor links rotatably coupled to a first anvil arm of the adjacent pair of anvil arms and a second link of the pair of scissor links rotatably coupled to a second anvil arm of the adjacent pair of anvil arms.

Wherein the first and second links of a respective pair of scissor links are rotatably coupled to a common mounting point on a respective carriage unit that is positioned between the first anvil arm and the second anvil arm.

Wherein, via the linkage system and the coupling of the first and second links of each respective pair of scissor links to a respective carriage unit, each anvil arm of the plurality of anvil arms remains centered between a respective pair of carriage units positioned on opposing sides thereof.

Wherein each of the plurality of anvil arms comprises a linear slide by which respective links of the linkage system are coupled thereto, the linear slide providing a radially inward and outward movement of a pivot point at which the links are coupled to the anvil arms.

Wherein the cutting system further comprises a belt drive that drives the anvil shaft to cause rotation of the anvil wheel; and a knife roll servo motor coupled to the knife roll to drive rotation of the knife roll; wherein the knife roll servo motor is separate from the belt drive, such that the knife roll is driven separately from the anvil wheel via camming of the knife roll servo motor.

Wherein the cutting system further comprises a lower cutter box assembly having tracks formed along a top surface thereof; an upper cutter box assembly positioned above the lower cutter box assembly and movable laterally relative to the lower cutter box assembly along the tracks; and an adjustment mechanism actuatable to cause lateral movement of the upper cutter box assembly along the tracks; wherein each of the anvil wheel and the knife roll are mounted on the upper cutter box assembly, such that lateral movement of the upper cutter box assembly causes the anvil wheel and the knife roll to move laterally relative to the transfer mechanism.

A human-machine interface (HMI) is configured to receive operator inputs directed to a reconfiguring of the transfer mechanism and/or the cutting system; and generate commands responsive to the operator inputs that cause one or more operational settings of the transfer mechanism and/or the cutting system to be modified; wherein the one or more operational settings comprise at least one of a rotational speed of the drive shaft, a pitch between adjacent pucks, a speed of the plurality of servo motors, a lateral positioning of the anvil wheel and the knife roll, a rotational speed of the anvil shaft, and a speed of the knife roll servo motor.

A slip ring positioned on the drive shaft, and wherein communications and/or power connections to the plurality of servo motors are routed through the slip ring.

Wherein the connecting arm comprises a curved, arcuate-shaped connecting arm.

Wherein the center plate comprises an annular rail structure positioned about an outer circumference thereof, the rail structure including pitch rails on which the plurality of carriage units is mounted to be movable therealong.

Wherein each of the plurality of carriage units comprises a chassis including a pair of frame members, with the frame members positioned on opposing sides of the rail structure; a plurality of rollers coupled to the chassis and in rolling engagement with an inner edge and outer edge of the pitch rails to secure the carriage unit to the rail structure and provide for translation of the carriage unit along the pitch rails; and a puck support positioned on the chassis and movable relative thereto via a mating of rail guides of the puck support with the pitch rails, the puck support oriented generally orthogonal to the pitch rails and comprising a puck mount on one end thereof that is configured to receive the puck.

Wherein the carriage unit comprises a drag link that couples the connecting arm to the chassis, with the drag link rotatably coupled to each of the connecting arm and to a mount on one of the frame members of the pair of frame members; and wherein the drag link transforms rotational motion of the servo motor and the connecting arm into a translation of the carriage unit along the pitch rails.

A barrel cam stationarily situated about the transfer axis, the barrel cam having a spin cam race formed around an outer circumference thereof; and wherein each of the plurality of carriage units comprises a spin cam follower positioned on another end of the puck support, the spin cam follower in sliding or rolling communication with the spin cam race to spin the puck at least partially about a spin axis of the respective puck that is at least substantially perpendicular to the transfer axis, so as to reorient the article carried on the puck.

A pick-and-place system comprising a stationary vacuum manifold positioned adjacent the center plate and configured to transfer a vacuum from a vacuum source to an interior volume of the center plate; and a plurality of vacuum tubes coupled to the center plate, with one or more vacuum tubes of the plurality of vacuum tubes fluidly connecting the interior volume of the center plate with a respective carriage unit of the plurality of carriage units to transfer the vacuum thereto; wherein each vacuum tube is rotatably coupled to the center plate to swivel relative thereto, and wherein a length and/or configuration of each vacuum tube is variable to accommodate movement of a respective carriage unit along the rail structure.

A plurality of motor drives secured on the center plate and operably connected to the plurality of servo motors to control operation thereof; and a human-machine interface (HMI) in communication with the plurality of motor drives to provide commands thereto, the HMI configured to: receive operator inputs directed to a reconfiguring of the pick-and-place system; and provide commands to the plurality of motor drives to cam the plurality of servo motors according to a pre-determined velocity profile associated with the operator inputs and thereby alter positioning of the plurality of carriage units with respect to the center plate along the at least the portion of the transfer path.

In an embodiment that is not in accordance with the claims wherein the anvil wheel comprises a linkage system that mechanically couples the plurality of anvil arms together, the linkage system including a pair of scissor links provided between each adjacent pair of anvil arms of the plurality of anvil arms, with a first link of the pair of scissor links rotatably coupled to a first anvil arm of the adjacent pair of anvil arms and a second link of the pair of scissor links rotatably coupled to a second anvil arm of the adjacent pair of anvil arms.

In an embodiment that is not in accordance with the claims wherein the first and second links of a respective pair of scissor links are rotatably coupled to a common mounting point on a chassis of a respective carriage unit that is positioned between the first anvil arm and the second anvil arm.

In an embodiment that is not in accordance with the claims wherein, via the linkage system and the coupling of the first and second links of each respective pair of scissor links to the chassis of a respective carriage unit, each anvil arm of the plurality of anvil arms remains centered between a respective pair of carriage units positioned on opposing sides thereof.

In an embodiment that is not in accordance with the claims wherein each of the plurality of anvil arms comprises a linear slide by which respective links of the linkage system are coupled thereto, the linear slide providing a radially inward and outward movement of a pivot point at which the links are coupled to the anvil arms.

In an embodiment that is not in accordance with the claims wherein the cutting system further comprises a belt drive that drives the anvil shaft to cause rotation of the anvil wheel; and a knife roll servo motor coupled to the knife roll to drive rotation of the knife roll; wherein the servo motor is separate from the belt drive, such that the knife roll is driven separately from the anvil wheel.

In an embodiment that is not in accordance with the claims wherein the cutting system further comprises a lower cutter box assembly having tracks formed along a top surface thereof; an upper cutter box assembly positioned above the lower cutter box assembly and movable laterally relative to the lower cutter box assembly along the tracks; and an adjustment mechanism actuatable to cause lateral movement of the upper cutter box assembly along the tracks; wherein each of the anvil wheel and the knife roll are mounted on the upper cutter box assembly, such that lateral movement of the upper cutter box assembly causes the anvil wheel and the knife roll to move laterally relative to the transfer mechanism.

In an embodiment that is not in accordance with the claims wherein the transfer mechanism comprises a plurality of servo motors mounted to the center plate, with each servo motor operably connected to a respective carriage unit of the plurality of carriage units via a connecting arm, and with each of the plurality of servo motor operable, via camming thereof, to alter positioning of its respective carriage unit with respect to the center plate by circumferentially displacing the carriage unit along the transfer path.

In an embodiment in accordance with the claims wherein the mounting structure comprises a center plate and, wherein mounting the plurality of carriage units comprises mounting the plurality of carriage units onto an annular rail structure positioned about an outer circumference of the center plate, the rail structure including pitch rails on which the plurality of carriage units is mounted to be movable therealong.

The method further comprising coupling a respective puck to each of the plurality of carriage units to provide pucks that accommodate a desired article size that is to be produced by the cutting and transfer apparatus.

The method further comprising providing inputs to the cutting and transfer apparatus from a human-machine interface (HMI) indicative of a product size to be produced by the cutting and transfer apparatus; and positioning the cutter mechanism relative to the transfer mechanism based on the inputs from the HMI, wherein positioning the cutter mechanism comprises positioning an anvil wheel and a knife roll of the cutter mechanism relative to the transfer mechanism based on the inputs from the HMI to cut the incoming web of material at a cutting location.

The method further comprising controlling a rotational speed of the drive shaft and the anvil wheel based on the inputs from the HMI, the rotational speed of the drive shaft and the rotational speed of the anvil wheel being kept equal; controlling a rotational speed of the servo motors based on the inputs from the HMI; and controlling a rotational speed of the knife roll, via camming of a knife roll servo motor operatively connected to the knife roll, based on the inputs from the HMI.

A method for initiating product changeover in a web transfer apparatus that is not in accordance with the claims is described, the transfer apparatus including a puck wheel and an anvil wheel driven by at least one drive shaft, a plurality of carriage units operatively coupled along a circumference of the puck wheel, each carriage unit including an interchangeable puck, the anvil wheel having a plurality of anvil arms pivotably connected to an anvil hub and extending radially outward from the anvil hub, a pair of scissor links pivotally coupled between each adjacent pair of anvil arms, each pair of scissor links pivotally coupled to a respective carriage unit at a common center point, and a rotating blade in cooperation with the anvil arms, the method comprising optionally removing each puck having a first size from its corresponding carriage unit; optionally fastening a puck having a second size to each corresponding carriage unit; providing a human-machine interface (HMI) to perform the actions of: modifying a rotational speed of the at least one drive shaft to modify the velocity of the puck wheel and anvil wheel, the rotational speed proportional to a throughput of discrete articles cut from a continuous web of material provided to the puck wheel; accelerating a selected carriage unit to linearly displace the selected carriage unit from a first position to a second position along a portion of the circumference of the puck wheel, wherein displacement of the selected carriage unit changes an angular position of corresponding anvil arms adjacent the selected carriage unit; adjusting a rotational speed and angular position of the rotating blade so that the rotating blade meets a distal end of a corresponding anvil arm, and cuts the continuous web of material disposed between the rotating blade and the distal end of the corresponding anvil arm; and deaccelerating a selected carriage unit to linearly displace the selected carriage unit from the second position to the first position.

Wherein the step of removing each puck of the first size and attaching each puck of the second size is performed by a robotic mechanism or by a human.

Wherein the HMI is a computer, controller, wireless communication device, or a laptop computer.

Further including providing a servo motor to control the acceleration and deceleration of each carriage unit.

Further including providing a servo motor to control the rotational speed and angular position of the rotating blade.

Wherein the first position of the selected carriage unit along a portion of the circumference of the puck wheel reflects an equal interspacing between adjacent carriage units proximal to a product receiving surface.

Wherein the second position of the selected carriage unit the along a portion of the circumference of the puck wheel reflects a desired interspacing of adjacent carriage units proximal to the rotating blade.

Further including coupling a surface portion of each puck to a vacuum source to selectively carry the discrete articles cut from the continuous web of material.

Including providing each carriage unit with a quick connector engagement structure or fastenerless attachment to facilitate efficient swapping out of each puck on its corresponding carriage unit.

Including controlling a linear position of the rotating blade to move the rotating blade radially outwardly and radially inwardly relative to a distal end of a selected anvil arm, the selected anvil arm in radial alignment with the rotating blade.

Further including providing a servo motor or a stepper motor to control the linear position of the rotating blade.

Further including maintaining a fixed rotational velocity of the puck wheel relative to the anvil wheel via a belt drive coupling a drive shaft fixedly mounted to the puck wheel to a drive shaft fixedly mounted to the anvil wheel.

Further including adjusting the servo motor to linearly displace selected carriages unit along the circumference of the puck wheel to achieve a predetermined circumferential spacing between adjacent carriage units.

Further including spinning a puck of a selected carriage unit along a spin axis prior to positioning a selected carriage unit proximal to a product receiving surface.

Further including providing a servo motor or a barrel cam arrangement to spin the puck.

A method for initiating product changeover in a transfer apparatus in accordance with the invention, the transfer apparatus including a puck wheel and an anvil wheel driven by at least one drive shaft, a plurality of carriage units operatively coupled along a circumference of the puck wheel, each carriage unit including an interchangeable puck, the anvil wheel having a plurality of anvil arms pivotably connected to an anvil hub and extending radially outward from the anvil hub, and a rotating blade in cooperation with the anvil arms, the method comprising optionally removing each puck having a first size from its corresponding carriage unit; optionally attaching a puck having a second size to each corresponding carriage unit; sending commands, sequentially or simultaneously, to one or more controllers that control a drive shaft motor, a plurality of carriage unit motors, and a blade motor, to: modify a rotational speed of the drive shaft to modify the velocity of the puck wheel and anvil wheel; accelerate a selected carriage unit to linearly displace the selected carriage unit from a first position to a second position along a portion of the circumference of the puck wheel, wherein displacement of the selected carriage unit changes an angular position of corresponding anvil arms adjacent the selected carriage unit; adjust a rotational speed and angular position of the rotating blade so that the rotating blade meets a distal end of a corresponding anvil arm, and cuts a continuous web of material disposed between the rotating blade and the distal end of the corresponding anvil arm; and deaccelerate a selected carriage unit to linearly displace the selected carriage unit from the second position to the first position.

Wherein the step of optionally removing each puck of the first size and attaching each puck of the second size is performed by a robotic mechanism or by a human.

including providing each carriage unit with a quick connector engagement structure or fastenerless attachment to facilitate efficient swapping out of each puck on its corresponding carriage unit.

Further including providing a servo motor to control the acceleration of each carriage unit.

Further including providing a servo motor control a rotational speed and angular position of the rotating blade so that the rotating blade meets a distal end of a corresponding anvil arm, and cuts the continuous web of material disposed between the rotating blade and the distal end of the corresponding anvil arm.

A transfer apparatus configured to transfer and rotate a plurality of discrete articles cut from a continuous web of material that is not in accordance with the claims is described, the transfer apparatus comprising: a puck wheel configured to rotate about a rotational axis; a plurality of carriage units operatively coupled along a circumference of the puck wheel, each carriage unit having a puck that is selectively operable to carry the discrete article thereon; an anvil wheel having an anvil hub and a plurality of anvil arms pivotably connected to the anvil hub and extending radially outward from the anvil hub; a linkage system including a pair of scissor links pivotally coupled between each adjacent pair of anvil arms, and each pair of scissor links pivotally coupled to a respective carriage unit at a common center point; and wherein displacement of a selected carriage unit along the circumference of the puck wheel changes an angular position of adjacent anvil arms relative to the circumference of the puck wheel.

Including a plurality of servo motors, each operatively coupled to a respective carriage unit via a connecting arm, and configured to displace a corresponding carriage unit along a portion of the circumference of the puck wheel.

Wherein the puck wheel comprises: a center plate mounted to a drive shaft and configured to rotate therewith about the rotational axis; an annular rail disposed on an outer circumferential portion of the center plate; and wherein the plurality of carriage units are mounted on the annular rail and are movable along a portion of the annular rail.

including a cutting apparatus operatively coupled to the transfer apparatus and configured to cut the continuous web of material into the plurality of discrete articles.

Wherein the cutting apparatus comprises: a knife roller having at least one knife mounted thereon; an anvil positioned at an end of each of the plurality of anvil arms; and wherein the anvils periodically cooperate with the at least one knife to cut an incoming web of material during rotation of the puck wheel and the anvil wheel.

Further including: a vacuum manifold operatively coupled to the center plate and configured to transfer a vacuum from a vacuum source to an interior volume of the center plate; and a tubular arrangement in fluid communication between the interior volume of the center plate and each respective carriage unit to transfer vacuum to the carriage unit.

Wherein the tubular arrangement comprises a plurality of telescoping tubes.

Wherein each of the plurality of anvil arms includes a distal linear slide portion to which endpoints of two scissor links are coupled at a pivot point, the linear slide providing a radially inward and radially outward movement of the pivot point to accommodate displacement of a selected carriage unit along the circumference of the puck wheel.

A cutting and transfer system that is not in accordance with the claims is described comprising: a cutting apparatus configured to cut an incoming continuous web of material into a plurality of discrete articles; and a transfer apparatus operably coupled to the cutting apparatus and configured to transfer and rotate the plurality of discrete articles from at least a web receiving location to an article placement location, the transfer apparatus comprising: a puck wheel configured to rotate about a rotational axis; a plurality of carriage units operatively coupled along a circumference of the puck wheel, each carriage unit having a puck that is selectively operable to carry the discrete article thereon, wherein each carriage unit is independently displaceable along a portion of the circumference of the puck wheel; an anvil wheel having an anvil hub and a plurality of anvil arms pivotably connected to the anvil hub and extending radially outward from the anvil hub; a pair of scissor links pivotally coupled between each adjacent pair of anvil arms at opposite ends thereof, and pivotally coupled to a respective carriage unit at a common center point; and wherein displacement of a selected carriage unit along the circumference of the puck wheel changes an angular position of adjacent anvil arms relative to the circumference of the puck wheel via the scissor links.

Including a plurality of servo motors, each servo motor operatively coupled to a respective carriage unit via a connecting arm, and configured to translate a corresponding carriage unit along the portion of the circumference of the puck wheel.

Wherein the puck wheel comprises: a center plate mounted to a drive shaft and configured to rotate therewith about the rotational axis; and an annular rail disposed along an outer circumference of the center plate, wherein the plurality of carriage units are mounted on the annular rail and are movable along a portion of the annular rail.

Wherein the cutting apparatus comprises: a knife roller having at least one knife mounted thereon; an anvil positioned at a distal end of each of the plurality of anvil arms; and wherein the anvils periodically cooperate with the at least one knife to cut the incoming web of material during rotation of the puck wheel and the anvil wheel.

Further including: a vacuum manifold operatively coupled to the center plate and configured to transfer a vacuum from a vacuum source to an interior volume of the center plate; and a tubular arrangement in fluid communication between the interior volume of the center plate and each respective carriage unit to transfer the vacuum to the carriage unit.

Wherein the tubular arrangement comprises a plurality of telescoping tubes.

Wherein each of the plurality of anvil arms comprises a linear slide portion to which endpoints of two scissor links are coupled at a pivot point, the linear slide providing a radially inward and radially outward movement of the pivot point to accommodate the displacement of a selected carriage unit along the circumference of the puck wheel.

A cutting and transfer system that is not in accordance with the claims is described comprising: a cutting apparatus configured to cut an incoming continuous web of material into a plurality of discrete articles; a transfer apparatus operably coupled to the cutting apparatus and configured to transfer and rotate the plurality of discrete articles from at least a web receiving location to an article placement location, the transfer apparatus comprising: a puck wheel; an anvil wheel; a drive shaft configured to rotate the puck wheel and the anvil wheel about a rotational axis; the puck wheel further including: a center plate; an annular rail disposed on an outer circumference of the center plate; a plurality of carriage units operatively mounted along the annular rail and independently movable along a portion of the annular rail; a plurality of pucks, each puck coupled to a corresponding carriage unit, each puck selectively operable to carry a discrete article thereon; a plurality of servo motors, each operatively coupled to a respective carriage unit via a connecting arm, and configured to displace a corresponding carriage unit along a portion of the circumference of the puck wheel; the anvil wheel further including: an anvil hub; a plurality of anvil arms pivotably connected to the anvil hub and extending radially outward from the anvil hub; a pair of scissor links pivotally coupled between each adjacent pair of anvil arms at opposite ends thereof, and pivotally coupled to a respective carriage unit at a common center point; and wherein displacement of a selected carriage unit along the annular rail changes an angular position of adjacent anvil arms relative to the circumference of the puck wheel via movement of the scissor links.

Wherein the anvil wheel is mounted to an anvil drive shaft wherein the puck wheel is mounted to a puck drive shaft; and wherein the puck wheel and the anvil wheel are driven by the drive shaft.

Wherein the cutting apparatus comprises: a knife roller having at least one knife mounted thereon; an anvil positioned at an end of each of the plurality of anvil arms; and wherein the anvils periodically cooperate with the at least one knife to cut the incoming web of material during rotation of the puck wheel and the anvil wheel.

Further including: a vacuum manifold operatively coupled to the center plate and configured to transfer a vacuum from a vacuum source to an interior volume of the center plate; and a tubular arrangement in fluid communication with an interior volume of the center plate and with each respective carriage unit to transfer the vacuum to each carriage unit.

Wherein each carriage unit is configured to rotate the puck about a spin axis using a servo motor or a mechanical cam arrangement operatively coupled to a circumferential portion of the puck wheel.

These and other advantages and features will be more readily understood from the following detailed description of preferred embodiments of the invention that is provided in connection with the accompanying drawings.

### BRIEF DESCRIPTION OF THE DRAWINGS

The drawings illustrate embodiments presently contemplated for carrying out the invention.

In the drawings:
FIG. 1 is a front perspective view of a configurable cutting and transfer apparatus, according to an embodiment of the invention.
FIG. 2 is a rear perspective view of the apparatus of FIG. 1.
FIG. 3 is a front elevational view of the apparatus of FIG. 1 with a cutter box assembly removed.
FIG. 4 is a front elevational view of an anvil wheel and arrangement of carriage units of the apparatus of FIG. 1 in isolation.
FIG. 5 is a detailed perspective view of a carriage unit mounted on a center plate of a transfer mechanism of the apparatus of FIG. 1.
FIG. 6 is a rear perspective view of a plurality of servo motors mounted on a center plate of a transfer mechanism of the apparatus of FIG. 1.
FIG. 7 is a detailed perspective view of a carriage unit engaging a spin cam race of a barrel cam of the apparatus of FIG. 1.
FIGS. 8 and 9 are top perspective views of a carriage unit included in the apparatus of FIG. 1, according to an embodiment of the invention.
FIG. 10 is a perspective view of the carriage unit of FIGS. 8 and 9 showing it coupled to a transfer mechanism and cutting system of the apparatus of FIG. 1.
FIG. 11A is a flowchart illustrating a process for reconfiguring a cutting a transfer apparatus, according to an embodiment of the invention.
FIG. 11B is a flowchart illustrating a process for reconfiguring a cutting a transfer apparatus, according to another embodiment of the invention.
FIG. 12 is a front elevation schematic representation of a first preferred velocity profile of a configurable cutting and transfer apparatus, according to an embodiment of the invention.
FIG. 13 is a graphical view of an exemplary velocity profile of FIG. 12.
FIG. 14 is a front elevation schematic representation of puck position changing relative to a transfer axis of rotation, the puck following the velocity profile of FIG. 12.
FIG. 15 is a simplified front elevation view of a configurable cutting and transfer apparatus in a first position, according to an embodiment of the invention.
FIG. 16 is a front elevation view of the apparatus of FIG. 15 in a second position, eliminating some detail to better illustrate functionality.
FIG. 17 is a front elevation view of the apparatus of FIG. 15 in a third position, eliminating some detail to better illustrate functionality.
FIG. 18 is a front elevation view of the apparatus of FIG. 15 in a fourth position, eliminating some detail to better illustrate functionality.
FIG. 19 is a front elevation view of the apparatus of FIG. 15 in a fifth position, eliminating some detail to better illustrate functionality.
FIG. 20 is a front elevation view of the apparatus of FIG. 15 in a sixth position, eliminating some detail to better illustrate functionality.
FIG. 21 is a front elevation view of the apparatus of FIG. 15 in a seventh position, eliminating some detail to better illustrate functionality.
FIG. 22 is a front elevation view of the apparatus of FIG. 15 in an eighth position, eliminating some detail to better illustrate functionality.
FIG. 23 is a graphical view of a velocity profile of a configurable cutting and transfer apparatus, according to another embodiment of the invention.
FIG. 24 is a graphical view of a velocity profile of a configurable cutting and transfer apparatus, according to another embodiment of the invention.
FIG. 25 is a graphical view of a velocity profile of a configurable cutting and transfer apparatus, according to another embodiment of the invention.
FIG. 26 is a front elevational view of a configurable cutting and transfer apparatus, according to another embodiment of the invention.
FIGS. 27 and 28 are perspective and front elevational views of a pick-and-place system, according to another embodiment of the invention.

### DETAILED DESCRIPTION

Embodiments of the invention are directed to a configurable cutting and transfer apparatus having a servo motor sub-system for facilitating the transfer of absorbent article inserts. Although the disclosure hereof is provided in sufficient detail to enable those skilled in the art to practice the invention, the physical embodiments herein disclosed merely exemplify the invention, which may be embodied in other specific structures. While the preferred embodiments have been described, the details may be changed without departing from the invention.

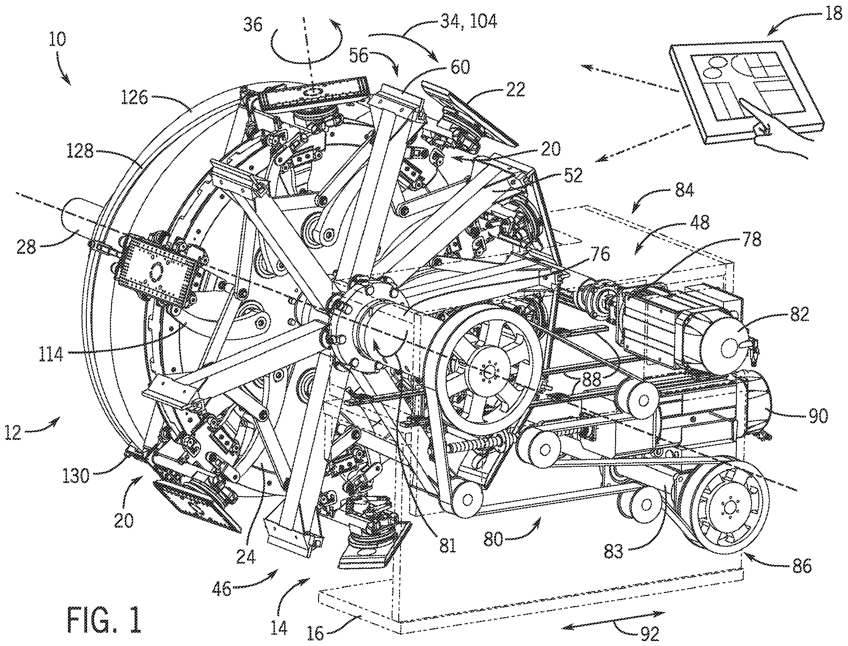

Referring to FIGS. 1-3, views of a cutting and transfer apparatus 10 (or "apparatus 10") are shown, respectively, according to an embodiment of the invention. The apparatus 10 preferably includes a transfer mechanism 12 and a cutting system 14, along with a frame 16 onto which the transfer mechanism 12 and cutting system 14 may be mounted. Operation of each of the transfer mechanism 12 and cutting system 14 in the apparatus 10 may be controlled via an associated human-machine interface (HMI) 18. According to one embodiment, and as shown in FIG. 1, the HMI 18 may be separate from the apparatus 10 and provided as part of a remote computing device such as a laptop computer or remotely located dedicated controller that communicates with the apparatus 10 via a web-based communication or other wireless communication protocol. According to another embodiment, the HMI 18 may be included as part of a control panel that is provided on the apparatus 10 to enable direct communication with the apparatus 10. In either embodiment, the HMI 18 may have a processor and memory (not shown) included therein that function to receive operator commands and perform control functions of the HMI 18 via executing one or more programs which may be contained within the memory, and with the memory contained therein stored values (such as the pre-determined product configuration settings for the apparatus 10) and programs for execution.

As will be explained in greater detail below, the apparatus 10 may be reconfigured to implement a different process flow and/or accommodate a different product type by modifying operational settings of the cutting system 14 and/or transfer mechanism 12 via operator inputs to the HMI 18 according to what is termed herein as a "push-button" change to the apparatus. The push-button change to the apparatus 10 provided via the HMI 18 enables at least some of the operational settings of the cutting system 14 and/or transfer mechanism 12 to be modified in an automated fashion responsive only to commands from the HMI 18, including embodiments where all operational settings of the cutting system 14 and/or transfer mechanism 12 are modified in an automated fashion and/or components are swapped out in an automated fashion (e.g., robotic changing of components). However, it is recognized that at least some manual reconfiguring of the apparatus 10 may be required even with the push button changes enabled by the HMI 18, with this concept explained in further detail below.

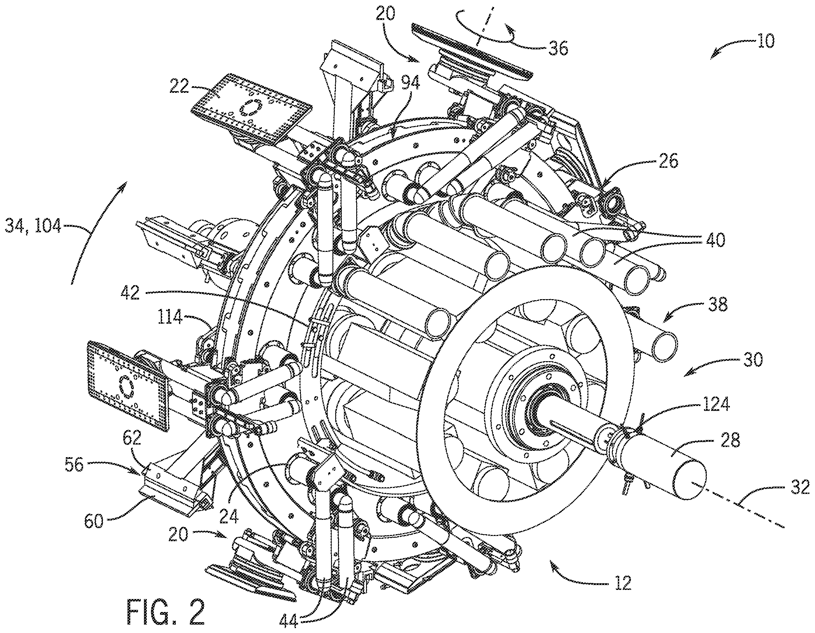

As best shown in FIG. 2, the transfer mechanism 12 includes a plurality of carriage units 20 each including a puck 22 that may be engaged and disengaged therefrom. The carriage units 20 are coupled to (and movable on) a mounting structure of the transfer mechanism 12 that, in an exemplary embodiment, is a center plate 24, with the carriage units 20 and center plate 24 collectively forming a puck wheel 26 of a desired configuration. In the illustrated embodiment, the puck wheel 26 includes a total of eight (8) carriage units 20 and pucks 22 positioned on the center plate 24, although it is recognized that more or less carriage units 20 and pucks 22 could be provided, such as nine (9) for example, in another non-limiting example.

The center plate 24 is fixedly coupled to a motor-driven shaft 28 that provides a substantially operationally constant rotational force to the center plate 24. The center plate 24 includes fastener holes (not shown) formed therethrough by which the center plate 24 is secured to the shaft 28, with the shaft 28 extending out from the center plate 24 on a drive side 30 of the apparatus 10. The center plate 24 - along with carriage units 20 and pucks 22 mounted thereto - is thus caused to rotate about a puck transfer axis 32 that is a major axis of rotation, so as to move the pucks about a transfer path 34. As used throughout the description of the preferred embodiment, "rotate" and its variants refer to the movement of an entire puck 22 (and carriage unit 20) about the transfer axis 32, while "spin" and its variants refer to the radial spin of a puck 22 about a puck spin axis 36, which is substantially perpendicular to the puck transfer axis 32, as will be explained further below.

Also positioned on the drive side 30 of the apparatus 10 is a vacuum system 38 that provides a vacuum to the individual carriage units 20 and pucks 22 of the transfer mechanism 12. A vacuum source (not shown) provides a vacuum to an arrangement of tubes 40 that feed into a stationary vacuum manifold 42 that is positioned adjacent the center plate 24 on the drive side 30. The stationary vacuum manifold 42 transfers a vacuum into an interior of the rotating center plate 24, with the vacuum then being transferred out of the center plate 24 and to the carriage units 20 via a plurality of telescoping tubes 44 coupled therebetween. The telescoping tubes 44 are coupled to the center plate 24 via rotatable bearings (not shown), such that the telescoping tubes 44 may swivel relative to the center plate 24. The swivel capability of the telescoping tubes 44, along with a telescopic construction that allows for the length thereof to increase/decrease, accommodates a movement of the carriage units 20 and pucks 22 relative to the center plate 24 (i.e., a displacement of the carriage units 20 and pucks 22 along the center plate) while still providing for a communication of the vacuum to the pucks 22, as will be described in more detail below.

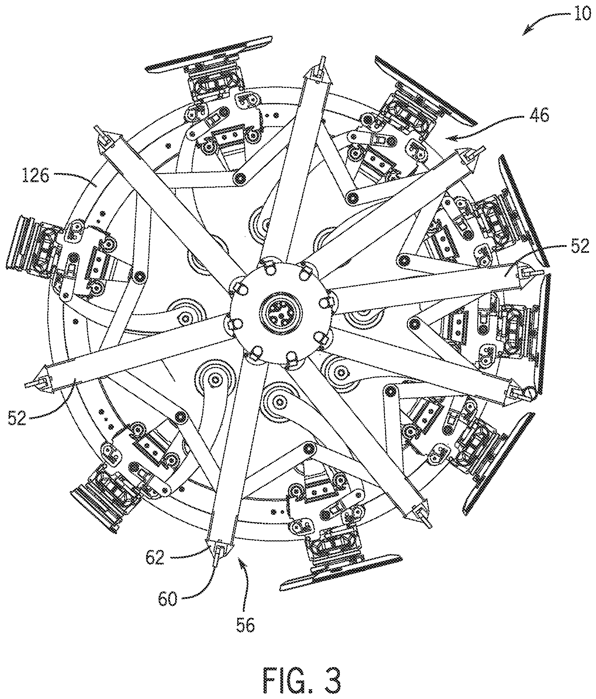

As best shown in FIGS. 1 and 3, the cutting system 14 preferably comprises an anvil wheel 46 and a knife roll 48 that interact with one another to cut discrete articles, for example absorbent pads or inserts, from a continuous web that is provided to the cutting and transfer apparatus 10. While the cutting system 14 is described herein as comprising an anvil wheel 46 and knife roll 48, it is recognized that these components could be reversed - with a knife wheel and anvil roll being utilized as compared to the illustrated embodiment. Operation of the knife wheel and anvil roll would be substantially similar to the operation of the anvil wheel 46 and knife roll 48 that is set forth here below.

The anvil wheel 46 includes a central anvil hub 50 from which a plurality of anvil arms 52 extend radially outward about a 360° range of the anvil wheel 46. Each of the anvil arms 52 is coupled to the anvil hub 50 via a pivot connection 54, with the pivot connections 54 spaced equidistant from one another about the anvil hub 50. Each of the anvil arms 52 includes an anvil 56 positioned at the end thereof opposite the pivot connection 54. The anvils 56 are configured to interact with one or more knife blades 58 on the knife roll 48 to cut a material when a respective anvil 56 is positioned at a cutting location adjacent to a knife blade 58. According to one embodiment, the anvils 56 comprise carbide inserts 60 held in place with a wedge block 62. Anvils 56 may alternatively be made of cast iron or other appropriate materials. The number of anvils 56 (and anvil arms 52) on the anvil wheel 46 matches the number of pucks 22 provided on the transfer mechanism 12, with the anvils 56 and anvil arms 52 arranged relative to the pucks 22 such that an anvil 56 and anvil arm 52 is positioned between each adjacent pair of pucks 22.

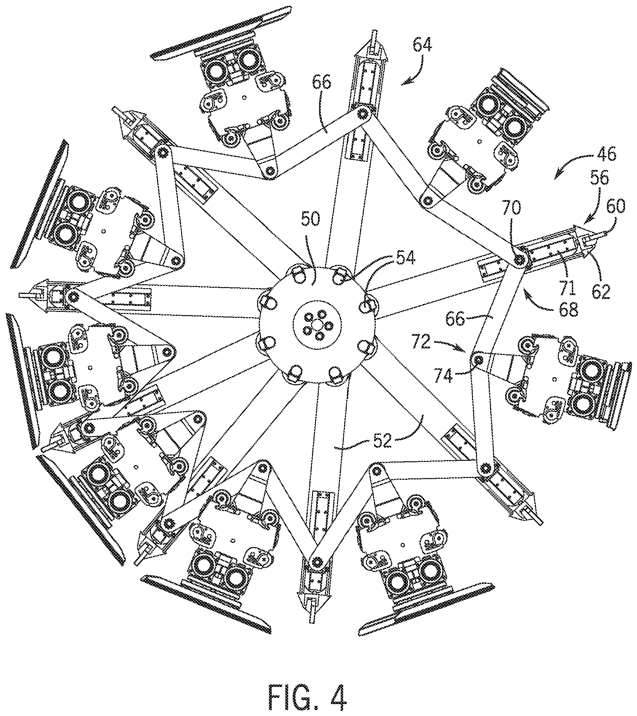

As shown in FIG. 1 and now also in FIG. 4, which shows a view of the anvil wheel 46 (and carriage units 20 and pucks 22) from the drive side 30 of the apparatus 10, the anvil wheel 46 further includes a linkage system 64 that mechanically couples the anvil arms 52 together. A pair of links (i.e., "scissor links") 66 is provided between each adjacent pair of anvil arms 52 of the anvil wheel 46, with a first end 68 of each link 66 coupled to an arm link mount 70 on a respective anvil arm 52 of the adjacent pair of anvil arms 52 and with a second end 72 of each link 66 coupled to a common mount 74 that is provided on a carriage unit 20. The coupling at each of the first and second ends 68, 72 of the links 66 to the arm link mount 70 on each anvil arm 52 and to the mount 74 on each carriage unit 20 is a pivotable coupling that allows the links 66 to rotate relative to each other and relative to the anvil arms 52 and the carriage unit 20. As will be described in further detail later, the positioning of each anvil arm 52 can therefore be controlled during operation of the apparatus 10 based, in part, on the positioning of each carriage unit 20 and associated puck 22 that are positioned on opposing sides thereof, with the anvil 56 and anvil arm 52 kept centered between its adjacent pucks 22 via the coupling of the anvil arm 52 to two adjacent carriage units 20 by the links 66.

As shown in FIG. 4, according to one embodiment, the arm link mount 70 on each anvil arm 52 is included on a linear slide 71 that allows the mount 70 to move radially inward and outward along the anvil arm 52. The positioning of the arm link mount 70 on the linear slide 71 provides a pivot point adjustment on the anvil arm 52 to accommodate different puck 22 (insert) sizes on the transfer mechanism 12, providing greater flexibility to the overall apparatus 10.

Regarding the cutting system 14, each of the anvil wheel 46 and knife roll 48 is coupled to and rotates on a respective shaft 76, 78, as shown in FIG. 1. The hub 50 of the anvil wheel 46 is mounted on an anvil shaft 76 that, in one embodiment, is a belt-driven shaft (via belt drive 80) that is driven in a 1:1 ratio to the puck wheel 26 via a jackshaft 83 that ties operation of the anvil wheel 46 together with operation of the puck wheel 26 of the transfer mechanism 12. The anvil shaft 76 rotates about an anvil axis 81 that may be aligned with the shaft 28 that that drives the transfer mechanism 12 (i.e., aligned with the transfer axis 32) or might be slightly laterally offset therefrom. In the illustrated embodiment, the knife roll 48 is mounted on a drive roll shaft 78 and is separately driven by a drive roll servo motor 82, although it is recognized that the knife roll 48 could be tied to the anvil drive (i.e., belt drive 80 drives both anvil shaft 76 and drive roll shaft 78). Each of the anvil wheel 46 and knife roll 48, as well as their respective shaft and drive mechanisms (i.e., belt drive 80 and servo motor 82) are collectively included as part of an upper cutter box assembly 84 of the cutting system 14. The upper cutter box assembly 84 is mounted on a stationary lower cuter box assembly 86. The upper cutter box assembly 84 is movable laterally relative to the lower cutter box assembly 86 via a pair of tracks 88 provided on a top surface of the lower cutter box assembly 86, with a manual hand crank or servo-driven lead screw adjustment 90 providing translation of the upper cutter box assembly 84 along the tracks 88. The upper cutter box assembly 84 may linearly translate along the tracks 88 in a direction 92 so as to provide for movement of the anvil wheel 46 and knife roll 48 relative to the transfer mechanism 12. As will be explained in greater detail below, when it is desirable to reconfigure the cutting and transfer apparatus 10 in order to implement a different process flow and/or accommodate a different product type or size, the upper cutter box assembly 84 may be moved along the tracks 88 to properly position the anvil wheel 46 and knife roll 48 relative to the transfer mechanism 12 and thereby maintain a proper position of the components for cutting the incoming continuous web of material provided to the apparatus 10.

In operation, the apparatus 10 receives a continuous web from a source and the web is brought into contact with a puck 22. One of anvils 56 is then caused to rotate into position so as to be aligned with a knife blade 58 on the knife roll 48 and cooperate therewith (i.e., come into contact with) to cut the web proximate a leading edge of the puck 22. After receipt of the web and the cut made near the leading edge, the puck 22 proceeds to travel along the transfer path 34 and past the knife roll 48, at which point the next anvil 56 on anvil wheel 46 rotates into position to cooperate with a knife blade 58 to cut the web proximate the trailing edge of the puck 22 to cut a discrete section from the web, to form a discrete article such as an insert or pad. The section is held to the puck 22 by a vacuum and caused to rotate about the transfer path 34, as will be explained in greater detail below.

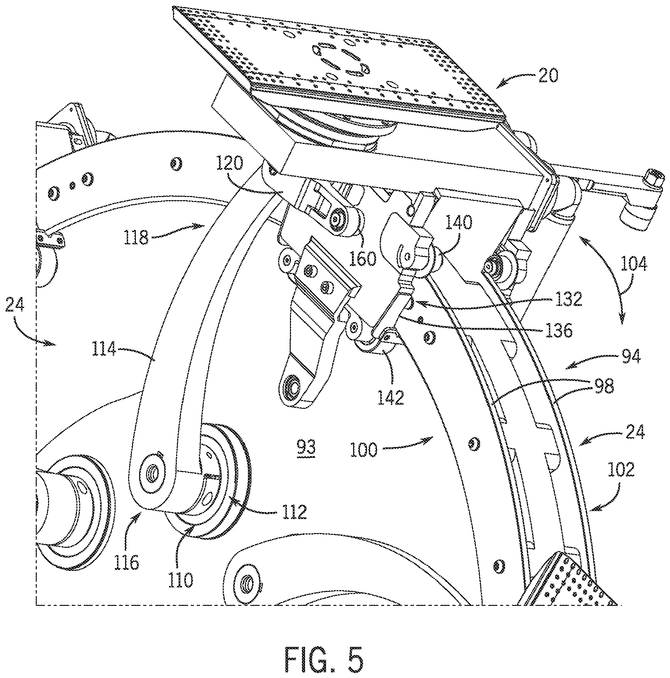

As indicated above, the carriage units 20 of the transfer mechanism 12 are coupled to the center plate 24 so as to be movable thereon. Specifically, the center plate 24 includes a main body 93 having a rail structure 94 positioned thereon that, in an example embodiment, is positioned about the outer circumference thereof, as shown in FIG. 2 and now also in FIG. 5. The rail structure 94 is a ring-like structure formed of a pair of tracks or rails 96 (hereafter "pitch rails 96"), with each of the pitch rails 96 coupled to the main body 93 of the center plate 24 via an intermediate spacer member 98. Each of the pitch rails 96 is thus spaced apart from the main body 93 of the center plate 24 in a width-wise direction. As indicated above, each of the pitch rails 96 has an annular shape, such that both an inner edge or surface 100 of the pitch rails 96 and an outer edge or surface 102 of the pitch rails 96 function as tracks along which the carriage units 20 may translate, although it is recognized that the pitch rails 96 may be configured such that the carriage units 20 translate along only one of the inner edge/surface 100 or the outer edge/surface 102. The pitch rails 96 run parallel to a machine direction 104 in which transfer mechanism 12 rotates to allow for controlled circumferential displacement of the carriage units 20 and pucks 22 relative to the center plate 24 in the machine direction 104, thereby enabling a pitch change or altered circumferential spacing of the pucks 22. While displacement of the carriage units 20 and pucks 22 here is described herein as being "circumferential," it is to be understood that aspects and embodiments of the invention are not limited to applications utilizing a circular motion and that the transfer path 34 of the carriage units 20 and pucks 22 may be defined by the path of any supporting pitch rails 96 used, for example.

While not shown in FIGS. 2 and 5, it is recognized that a rail structure 94 of an alternative construction could be utilized, according to other embodiments. As an example, the rail structure 94 could be provided on opposing flat side surfaces of the center plate 24 rather than about the outer circumference of the center plate 24 (and facing radially outward). In such an embodiment, pitch rails 96 would be provided on each of the opposing flat side surfaces of the center plate 24 and face outwardly and away from their respective side surface. As with the previously described embodiment, the pitch rails 96 on the side surfaces of the center plate 24 would run parallel to a machine direction 104 in which the transfer mechanism 12 rotates to allow for controlled circumferential displacement of the carriage units 20 and pucks 22 relative to the center plate 24 in the machine direction 104, thereby enabling a pitch change or altered circumferential spacing of the pucks 22.

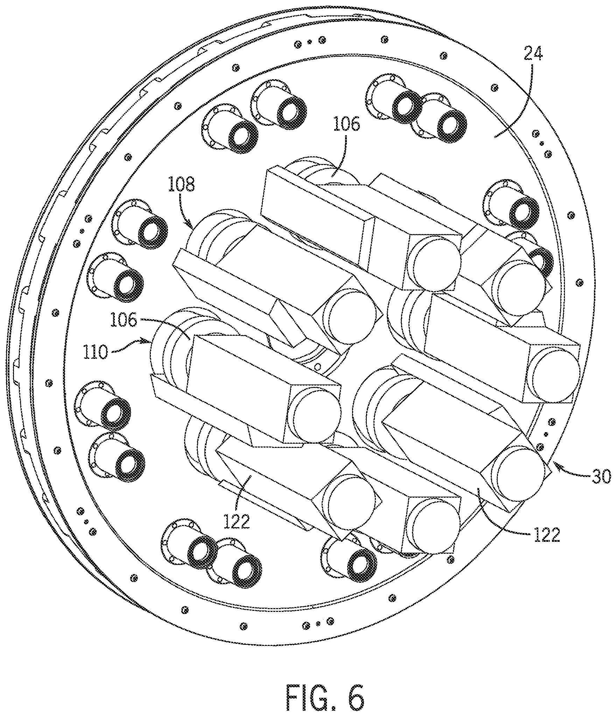

To facilitate circumferential displacement of the carriage units 20 and pucks 22 along the pitch rails 96, the transfer mechanism 12 includes a plurality of servo motors 106, as shown in FIG. 2 and now in FIG. 6, with it understood that the term "servo motor" as used herein may refer to a servo motor system including only a servo motor or to a servo motor and gearbox combination. A dedicated servo motor 106 is provided for each carriage unit 20 to enable independently controlled displacement of its carriage unit 20 (and puck 22) along the pitch rails 96. In one embodiment, the servo motors 106 are mounted on the drive side 30 of the center plate 24, with a plurality of openings 108 formed in the center plate 24 through which the servo motors 106 extend, such that an output end 110 (i.e., side that outputs a rotational power) of each servo motor 106 extends through to a cutter side 112 of the center plate 24 and apparatus 10.

As best shown in FIG. 5, a connecting arm 114 is coupled to the output end 110 of each servo motor 106 and is displaced (i.e., rotated) responsive to a rotational output of the servo motor 106. In the example implementation, the connecting arm 114 has a curved, arcuate-shaped profile, but in other implementations the connecting arm 114 could have a straight-arm configuration. A first end 116 of the connecting arm 114 is fixedly coupled to the output end 110 of the servo motor 106 and a second end 118 of the connecting arm 114 is rotatably coupled to a respective carriage unit 20 via a drag link 120. In operation, a servo motor 106 may rotate both clockwise and counterclockwise, such that the connecting arm 114 moves back and forth in a reciprocating, windshield wiper type pattern. Via this motion of the connecting arm 114, and via the drag link 120 rotatably coupling the connecting arm 114 to the carriage unit 20, rotational motion of a servo motor 106 may therefore be transformed into a linear motion (i.e., motion in the machine direction 104) that circumferentially displaces a respective carriage unit 20 along the pitch rails 96.

As shown in FIG. 6, each of the servo motors 106 may be controlled by a respective motor drive 122 or other control device that is configured to operate the servo motor 106 according to a desired velocity profile that is input to the motor drive 122 via commands from the HMI 18 (FIG. 1), such as receiving wireless control signals from the HMI 18, for example. In one embodiment, pre-programmed velocity profiles may be stored on the HMI 18 (i.e., in the memory of the HMI 18) associated with product configuration settings for the apparatus 10 for a product type/size to be produced, with these pre-programmed velocity profiles being transmitted to the motor drives 122. Based on the input velocity profile, the drives 122 may be programmed to operate the respective servo motors 106 at a desired speed based, in one example, on its circumferential position on the puck wheel 26. The drive 122 of each servo motor 106 would be programmed in an identical fashion (by inputs from the HMI 18), such that each servo motor 106 is operated in the same manner and according to the same velocity profile. An example operation of a servo motor 106 to control circumferential displacement of a puck 22 is provided in further detail below. In one embodiment, the drives 122 are positioned adjacent their respective servo motors 106 on the drive side 30 of the center plate 24, and communications and power connections may be routed to the drives 122 and servo motors 106 via use of a slip ring 124 (FIG. 2) positioned on the drive shaft 28, although it is recognized that a wireless communications and power transmission to the drives 122 and servo motors 106 may instead by utilized.

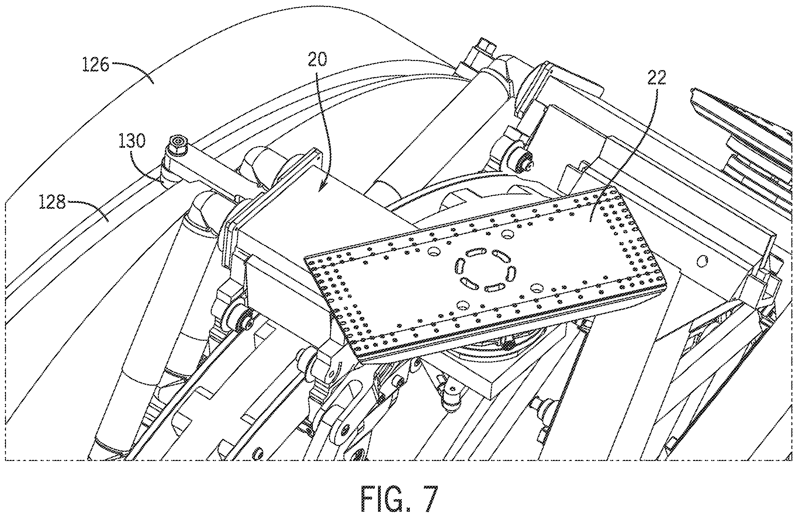

As previously indicated, in addition to the transfer mechanism 12 providing for a rotation of the carriage units 20 and pucks 22 about the transfer axis 32, the transfer mechanism 12 also provides for a radial spinning of each puck 22 about a puck spin axis 36. To facilitate this spinning of the pucks 22, the transfer mechanism 12 also includes a barrel cam 126 situated about the transfer axis 32 and positioned on the drive side 30 of the center plate 24, as shown in FIGS. 1 and 3 and now also in FIG. 7. The barrel cam 126 is preferably a stationary ring-shaped member having a spin cam race 128 provided around an outside edge/surface thereof. To achieve a desired spin of the pucks 22, a spin cam follower 130 of the carriage unit 20, which is preferably a roller bearing, is in sliding or rolling communication with the spin cam race 128. In the example implementation, spin cam race 128 is configured to provide a ninety-degree (90°) puck rotation, but it is recognized that configuration of the spin cam race 128 is generally determined by the desired spin angle of the puck 22. While the barrel cam 126 is shown and described herein as being constructed as cylindrically shaped barrel having a spin cam race 128 provided around an outside edge/surface thereof, it is recognized that the barrel cam 126 could instead be configured as an annular ring-shaped member having a spin cam race 128 provided around an inside edge/surface thereof, i.e., an inverted barrel cam ring, according to another embodiment.

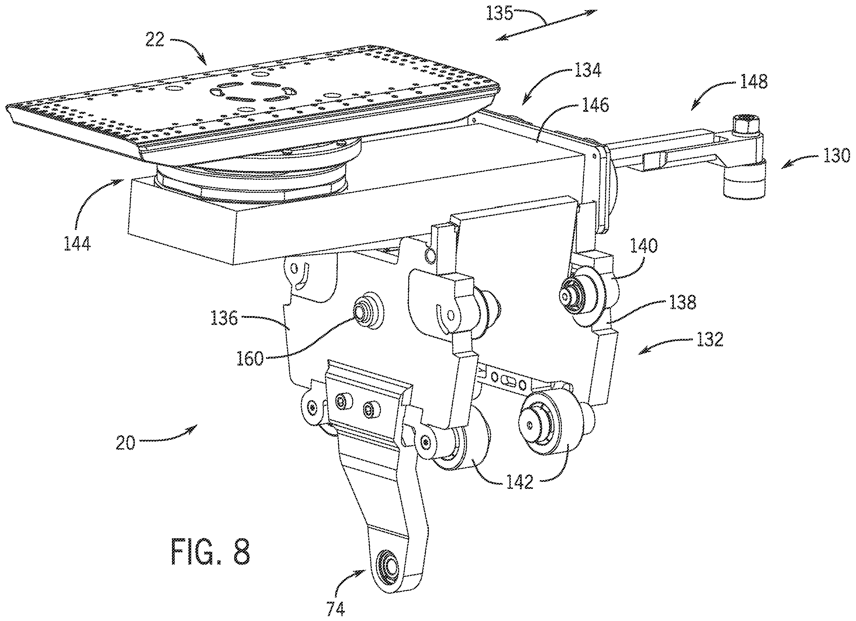

Referring still to FIGS. 1-7 and now also to FIGS. 8-11, a carriage unit 20 is shown in greater detail for purposes of better describing the structure and operation thereof that enables the rotation, circumferential displacement, and spinning thereof. The carriage unit 20 generally includes a chassis 132 and a puck support 134 that is positioned on the chassis 132. The chassis 132 generally secures the carriage unit 20 to the rail structure 94 of the center plate 24 and provides for circumferential displacement of the carriage unit 20 along the pitch rails 96. The chassis 132 includes a pair of chassis frame members 136, 138 joined together in a spaced apart arrangement so as to accommodate positioning of the rail structure 94 therebetween. A plurality of rollers 140, 142 is coupled to the chassis frame members 136, 138 in an arrangement that allows for the carriage unit 20 to be secured onto the rail structure 94 and provides for translation of the carriage unit 20 along the pitch rails 96. In one example, a pair of upper rollers 140 and a pair of lower rollers 142 are provided on each of the chassis frame members 136, 138, with the two lower rollers 142 engaging the inner edge or surface 100 of a respective pitch rail 96 and the two upper rollers 140 engaging the outer edge or surface 102 of a respective pitch rail 96.

According to another embodiment, and where the rail structure 94 is provided on opposing flat side surfaces of the center plate 24 as previously described, the arrangement and orientation of the rollers 140, 142 would be altered to allow for the carriage unit 20 to be properly secured onto the rail structure 94 and provide for translation of the carriage unit 20 along the pitch rails 96. That is, a pair of "first side" rollers and a pair of "second side" rollers would be provided on each of the chassis frame members 136, 138 to engage the respective pitch rails 96 provided on each of the opposing flat side surfaces of the center plate 24.

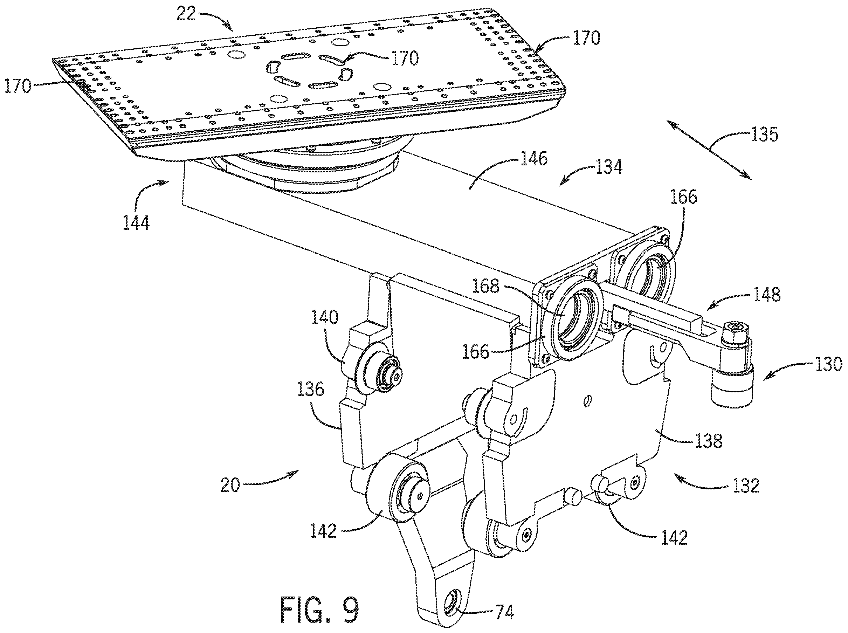

As best shown in FIGS. 8 and 9, the puck support 134 is secured on the chassis 132. The puck support 134 is oriented generally orthogonal to the chassis 132 (and to the rail structure 94) in a direction 135, with a puck mount 144 provided on one end of the puck support 134 (i.e., on the cutter side 112 of apparatus 10) and a spin cam follower 130 provided on the opposite end of the puck support 134. The puck support 134 includes a main body 146 that is fixedly coupled to the chassis 132 on a top or outward facing surface thereof, with the main body 146 spanning between the chassis frame members 136, 138, so as to be positioned over the rail structure 94 when the carriage unit 20 is mounted thereon.

As shown in FIGS. 8 and 9, the spin cam follower 130 of the puck support 134 is joined to the main body 146 via a take up frame 148. The take up frame 148 is coupled to the main body 146 and includes a plurality of mechanisms thereon that function to transfer movement of the spin cam follower 130 to the puck mount 144 and the puck 22 mounted thereon. That is, movement of the spin cam follower 130 is transferred to the puck mount 144 and puck 22 via the take up frame 148 and associated mechanisms to cause the puck 22 to rotate (i.e., spin) in a desired manner.

Also included in the carriage unit 20 are a number of mounting features that couple the carriage unit 20 to a respective servo motor 106 and to the anvil wheel 46 to provide interoperability therebetween. Specifically, the chassis frame member 136 on the cutter side 112 of the center plate 24 includes thereon a drag link mount 160 and a scissor link mount 74 by which the servo motor 106 and the anvil wheel 46 may be operatively connected to the carriage unit 20, as shown in FIG. 8. The drag link mount 160 may be provided as a cylindrical protrusion that is centered on the chassis frame member 136 in the machine direction 104. The scissor link mount 74 may be provided as a mounting hole formed in a protrusion extending outward from a bottom (i.e., radially-inward facing) edge of the chassis frame member 136.

The drag link mount 160 provides a connection point where the drag link 120 is coupled to the carriage unit 20, to thereby connect the carriage unit 20 to the servo motor 106 (via the connecting arm 114 and drag link 120), as shown in FIG. 8 and in FIG. 5. The drag link 120 is rotatably coupled to the drag link mount 160 (via a bearing attachment) such that the drag link 120 may rotate relative to the frame 136. In operation of the transfer mechanism 12, a respective servo motor 106 may be controlled to cause rotation of the connecting arm 114 coupled thereto. Via the connecting arm 114 and drag link 120, rotational motion of the servo motor 106 is transferred to the carriage unit 20, causing the chassis 132 to translate along rail structure 94 via the rollers 140, 142 sliding along the pitch rails 96 thereof. A carriage unit 20 may therefore be circumferentially displaced along the center plate 24 via operation of its respective servo motor 106 to control the pitch between the carriage unit 20 and its adjacent carriage units 20 of the puck wheel 26.

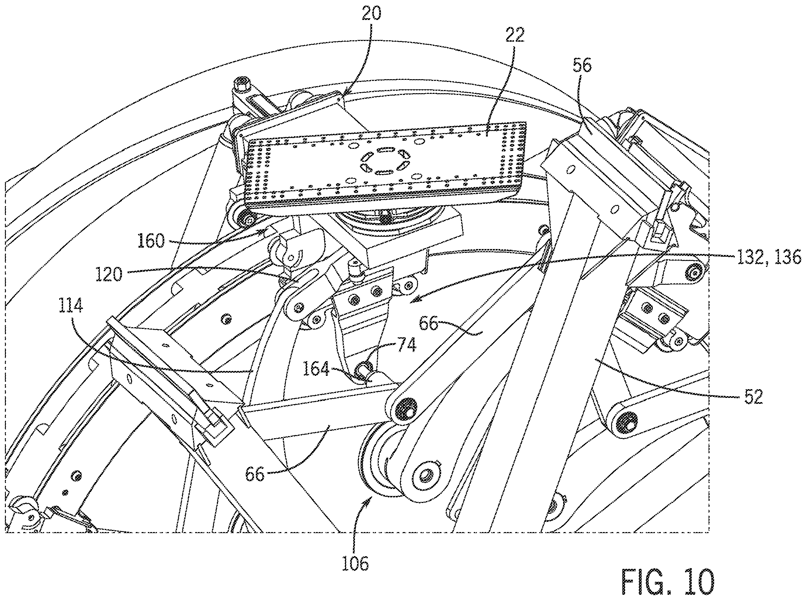

As shown in FIGS. 8 and 10, the scissor link mount 74 provides a connection point where a pair of scissor links 66 of the anvil wheel linkage system 64 is coupled to the carriage unit 20, with a cylindrical spacer 164 secured to the scissor link mount 74 and extending outwardly therefrom to provide a coupling point for the links 66 about which the links 66 may pivot. As previously described, in controlling the circumferential displacement of a carriage unit 20, the positioning of an anvil arm 52 and anvil 56 between two adjacent carriage units 20 may be controlled via a pair of scissor links 66 being provided between each adjacent pair of anvil arms 52 of the anvil wheel 46, with a first end 68 of each link 66 pivotably coupled to an arm link mount 70 on a respective anvil arm 52 of the adjacent pair of anvil arms 52 and with a second end 72 of each link 66 pivotably coupled to a common scissor link mount 74 (via spacer 164) that is provided on a carriage unit 20, as shown in FIG. 4. Each respective anvil arm 52 is thus coupled to two carriage units 20 via the links 66 that extend out therefrom in each of opposing directions and that are coupled to the scissor link mounts 74 of those carriage units 20. The connection of the anvil arm 52 to its two adjacent carriage units 20, via links 66, controls the positioning of that anvil arm 52 relative to the carriage units 20 - with the anvil arm 52 remaining centered between the carriage units 20 throughout operation of the transfer mechanism 12 regardless of the pitch/spacing between the carriage units 20 or the specific operating speed of the transfer mechanism 12. Additionally, the connection of the anvil arm 52 to its two adjacent carriage units 20 also ensures that each anvil 56 is matched to the speed of the incoming continuous web of material at cutting, as the speed of an anvil 56 at cutting is tied to the speed of the carriage units 20 and pucks 22 (which is in turn controlled by the servo motors 106) to which that anvil 56 (anvil arm 52) is connected.

As an example of controlling the positioning of an anvil arm 52 and anvil 56 between two adjacent carriage units 20, if a carriage unit 20 ahead of a respective anvil arm 52 (in the machine direction 104) is circumferentially displaced further forward by its associated servo motor 106, the scissor link 66 that couples the carriage unit 20 (at the scissor link mount 74 on chassis frame member 136) to the anvil arm 52 will cause the anvil arm 52 to angularly rotate forward toward the carriage unit 20. At the same time, the scissor link 66 that couples the anvil arm 52 to its trailing carriage unit 20 will limit the amount of forward angular rotation of the anvil arm 52. Accordingly, the positioning of the anvil arm 52 between its two adjacent carriage units 20 is maintained relatively constant, such that any contact/collision between the anvil arm 52 and its adjacent carriage units 20 is prevented.

Also included in carriage unit 20 are components that provide vacuum communication to the puck 22 to enable a cut pad or segment to be secured thereto. As best shown in FIG. 9, the main body 146 of the puck support 134 is formed as a semi-hollow member having vacuum channels 166 formed therein, with a pair of vacuum channels 166 being shown in the illustrated embodiment. The vacuum channels 166 extend for a majority of a length of the main body 146 and enable communication of a vacuum from a vacuum source (not shown) of apparatus 10 to the puck mount 144 and onto the puck 22, so as to enable discrete articles or pads to be held on the puck 22. When the carriage unit 20 is positioned on the center plate 24, openings 168 of the vacuum channels 166 are connected with the telescoping tubes 44 of the transfer mechanism 12 that communicate a vacuum thereto, as shown in FIG. 2. As previously described, a vacuum source provides a vacuum to the stationary vacuum manifold 42 that is positioned adjacent the center plate 24, with the vacuum transferring to an interior of the center plate 24 and then out through the telescoping tubes 44 and to the carriage unit 20.

With the carriage unit 20 fluidly connected to the vacuum source, the vacuum channels 166 communicate a vacuum to the puck mount 144 such that a vacuum is commutable therethrough. A vacuum is drawn through the vacuum channels 166 and provided to one or more vacuum zones 170 at the puck 22 (FIGS. 8 and 9), with the orientation of the puck 22 to the one or more zones 170 controlling whether the pucks 22 will pick-up or transfer (i.e., activate/deactivate the vacuum through the puck 22). According to one embodiment, each vacuum puck 22 has four vacuum zones 170 to assist in holding the pad/insert during pick-up and transfer. As the pucks 22 rotate, the leading and trailing vacuum zones 170 change. Pucks rotate 90 degrees after pick-up and then drop off the pad/insert to a receiving surface (not shown), such as a vacuum transfer roll.

Regarding the pucks, it is recognized that the pucks 22 are removable from the puck mounts 144 in order to allow for swapping/changing of the pucks 22 as desired to accommodate a specific set-up of the transfer mechanism 12. In mounting a puck 22 to a puck mount 144 of the carriage unit 20, one of various connection mechanisms may be utilized, including fastener-based attachments or fastenerless attachments. As one example, a puck 22 may be configured to engage a puck mount 144 according to a "quick connector" type engagement, e.g., a "hitch and receiver" type engagement. The structure of the puck 22 and mating thereof with the puck mount 144 allows for pucks 22 to be easily swapped out and exchanged on the carriage unit 20 based on the specific set-up of the transfer mechanism 12. Different size pucks 22 can be connected to the carriage units 20 to accommodate the cutting and transferring of articles of differing types and sizes.

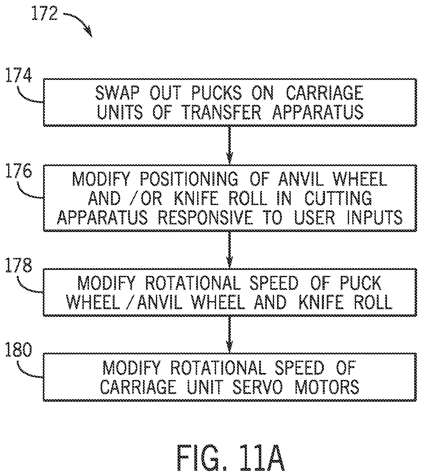

With reference again to FIGS. 1-10 and now also to FIG. 11A, a process 172 for configuring or reconfiguring the cutting and transfer apparatus 10 is now described according to an embodiment of the invention. While the process 172 is described below as a reconfiguring of an existing set-up of the apparatus 10, it is recognized that the process is also applicable to an initial configuring of the apparatus 10. With regard to the reconfiguring process 172, it is recognized that only some of the described steps may need to be performed when reconfiguring the apparatus 10, with the exact process that is implemented being determined by a revised new process flow and/or new product type/size to be processed by the apparatus 10. Thus, it is to be understood that the process 172 described here below is only meant to be an exemplary process and embodiments of the invention are not meant to be limited only to the described process.

When it is desirable to reconfigure the apparatus 10 in order to implement a different process flow and/or accommodate a different product type/size, a push-button change of the apparatus 10 may be implemented such as via the HMI 18 of the apparatus 10. Operator inputs may be provided to the HMI 18 to cause all or at least some of the operational settings of the cutting system 14 and/or transfer mechanism 12 to be modified in an automated fashion, thereby providing an automated or semi-automated change-over and reconfiguring of the apparatus 10.

As an optional first step in the reconfiguring process 172, the transfer mechanism 12 may be modified by attaching a swapping out the puck 22 on each carriage unit 20 to provide pucks 22 that are able to accommodate a new pad/product size that is to be produced by the updated cutting and transfer process, as indicated at step 174. As previously described, in one embodiment, the pucks 22 may be constructed to engage the puck mounts 144 via a quick connector type engagement to secure the puck 22 to the puck support 134 of a carriage unit 20. The structure of the pucks 22 and mating thereof with the puck mounts 144 allows for the pucks 22 to be easily swapped out and exchanged on the carriage unit 20 based on the specific set-up of the transfer mechanism 12. The swapping out and exchanging of different pucks 22 may be performed manually or, in an alternative embodiment, may be performed via the use of a robotic system or other change-over means. Resulting from the change of the pucks 22 mounted on the carriage units 20, the effective diameter of the overall puck wheel 26 may increase or decrease and the pitch to be maintained between adjacent pucks 22 during operation of the transfer mechanism 12 may also change.

As another step in the reconfiguring process 172, the cutting system 14 is modified responsive to inputs to the HMI 18 indicative of a product size change for upcoming use of the apparatus 10, as indicated at step 176. Specifically, positioning of the anvil wheel 46 and knife roll 48 is adjusted relative to the transfer mechanism 12 to account for the reconfiguring of the transfer mechanism 12, such as the increased/decreased effective diameter of the puck wheel 26. In one embodiment, the positioning of the anvil wheel 46 and knife roll 48 is automatically adjusted in response to an operator input provided to the HMI 18, with the servo-driven lead screw adjustment 90 operating to translate the upper cutter box assembly 84 laterally along the tracks 88 relative to the lower cutter box assembly 86. The anvil wheel 46 and knife roll 48 are thus repositioned laterally relative to the transfer mechanism 12 such that the anvils 56 are positioned correctly relative to the pucks 22 when cuts to the incoming continuous web of material are to be made at a cutting location between the anvils 56 and knife roll 48.

As part of the reconfiguring process 172 of the cutting system 14, operation of the knife roll 48 may also be modified to provide for proper interaction with the anvil wheel 46, as indicated at step 178. That is, with the reconfiguring of the transfer mechanism 12, the speed at which the puck wheel 26 is driven may be modified (via controlled operation of motor-driven shaft 28, also part of step 178), with the speed at which the anvil wheel 46 is driven thus also being modified (via controlled operation of belt drive 80) to maintain the 1:1 speed relationship between the anvil wheel 46 and puck wheel 26. To maintain proper phasing and operation of the knife roll 48 relative to the puck wheel 26 and anvil wheel 46, the speed of the knife roll 48 is thus also modified. Camming of the knife roll 48 is therefore performed during the reconfiguring process 172 via selective operation of the servo motor 82 that drives the drive roll shaft 78 and knife roll 48.

The push-button reconfiguring process 172 of the apparatus 10 also includes a reprogramming or changing of the control scheme for the servo motors 106, as indicated at step 180. That is, for any velocity or speed changes at which the puck wheel 26 is rotated, a corresponding change to the velocity or speed at which the servo motors 106 cause circumferential displacement of the carriage units 20 along the center plate 24 may also be made. The changing of the control scheme for the servo motors 106 may be easily accomplished via reprogramming of the associated drives 122, responsive to input commands to the HMI 18 from an operator of the apparatus 10.

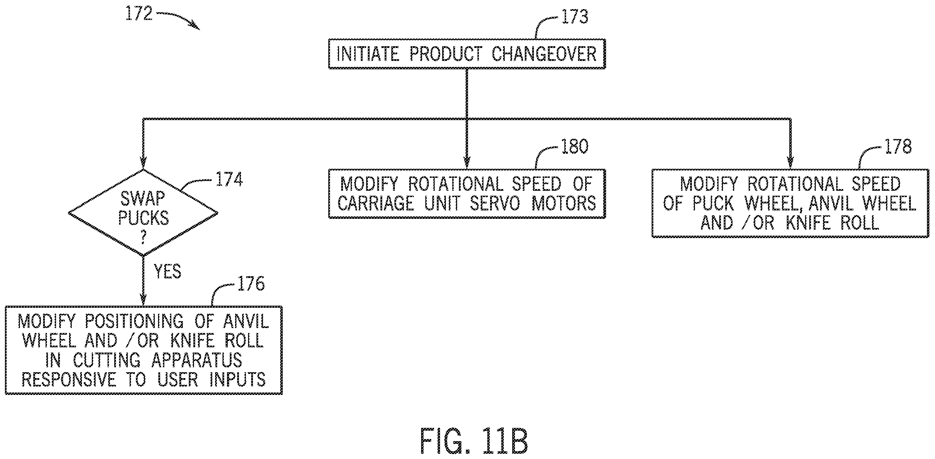

While process 172 is illustrated in FIG. 11A with steps 174-180 occurring sequentially, the steps of process 172 may occur in an alternative order to that shown in FIG. 11A. Furthermore, some of steps 174-180 may be carried out simultaneously. One alternative embodiment of process 172 is illustrated in FIG. 11B. At step 173 one or more operator inputs are provided to the HMI 18 during a product changeover sequence. These operator inputs may include, as non-limiting examples, a product size selection (e.g., Size 1, Size 2, Size 3, etc.), a product name selection (e.g., Product A, Product B, etc.), or specific product dimensions, such as product pitch and insert length as examples.

Responsive to the operator input, the HMI 18 optionally may generate an indication that pucks 22 must be swapped out as part of the product changeover process. If pucks 22 are to be swapped, the transfer mechanism 12 is modified by swapping out the puck 22 on each carriage unit 20 to provide pucks 22 that are able to accommodate a new pad/product size that is to be produced by the updated cutting and transfer process, as indicated at step 174. If pucks 22 are to be swapped, process 172 includes step 176 in which the positioning of the anvil wheel 46 and knife roll 48 is adjusted relative to the transfer mechanism 12 to account for the reconfiguring of the transfer mechanism 12. Step 176 may occur prior to or after the pucks 22 are swapped and may be done automatically based on commands triggered through the HMI 18 or manually in the same manners described with respect to FIG. 11A.

The operator input to the HMI 18 indicating a product changeover may also trigger a modification of the rotational speed of the transfer mechanism 12, anvil wheel 46, and/or knife roll 48, as indicated at optional step 178, in order to accommodate a change in machine speed (i.e., parts per minute) associated with the product changeover. Such modification(s) may be carried out automatically via programming changes and in the same manner described with respect to FIG. 11A without further operator input.

The operator input to the HMI 18 indicating a product changeover may also trigger an automatic reprogramming of the servo motor drives 122, as indicated at step 180, in order to modify the camming/rotational speed of the carriage units, in a similar manner as described with respect to FIG. 11A.

The push-button reconfiguring process 172 of the apparatus 10 as described above thus allows for the efficient processing of products of different types/sizes as compared to a previous apparatus set-ups and constructions, with the reconfiguration being performed in a semi-automatic fashion and without having to swap out the entire transfer mechanism 12 and/or cutting system 14. The reconfiguring process 172 may thus be performed in a quick and efficient manner where downtime of the cutting and transfer apparatus 10 is minimized.

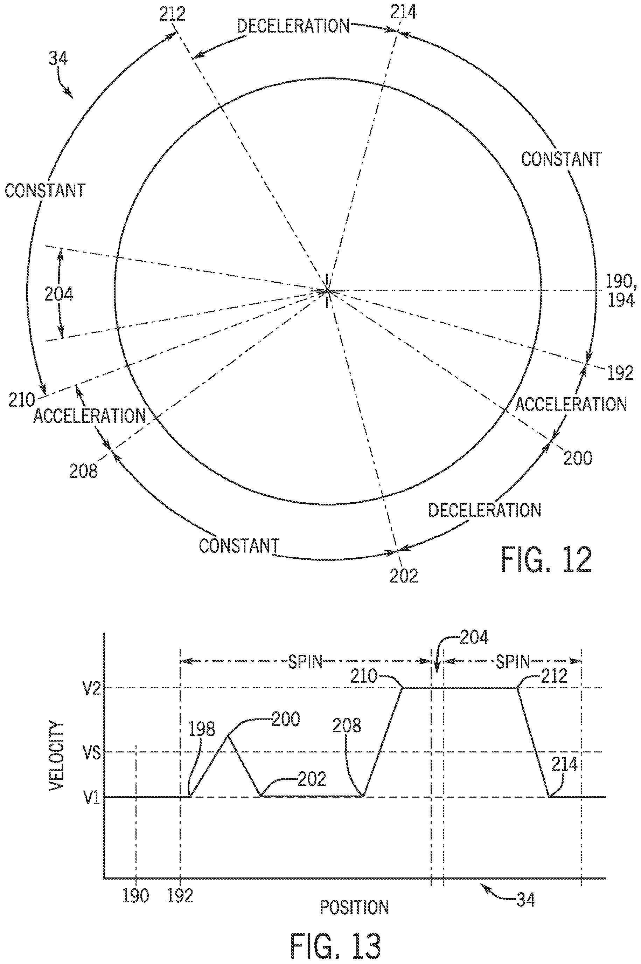

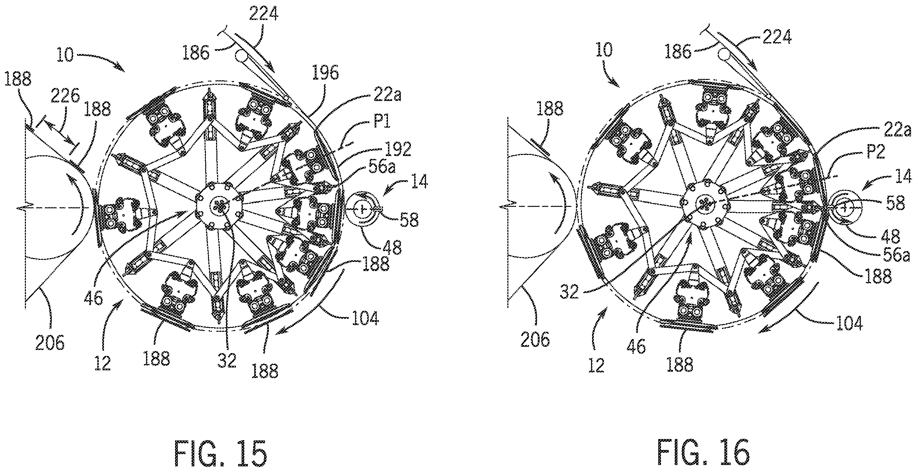

Referring now to FIGS. 12-22, an example operation of the configurable cutting and transfer apparatus 10 is described in greater detail for purposes of better illustrating embodiments of the invention. The apparatus 10 is illustrated with the transfer mechanism 12 including an arrangement of eight (8) pucks 22 thereon, but it is recognized that operation of the apparatus 10 would be the same with a greater or lesser number of pucks 22. Additionally, while operation of the apparatus 10 is described with reference to a single puck 22a and a single anvil 56a, it is to be understood that the operation of the remaining pucks 22 and anvils 56 is at least substantially similar. Furthermore, although the operation is described with reference, in FIGS. 15-22, to discrete puck positions, it is to be understood that the operation is preferably generally continuous. The discrete positions aid in illustrating the operations being performed. Still further, the apparatus 10 may be configured to rotate in a generally clockwise manner, as shown in FIGS. 15-22, or in a generally counterclockwise manner for an opposite-handed machine configuration.

Referring first to FIGS. 12-14, and with reference also to FIGS. 15-22, an exemplary puck velocity profile is depicted as each puck 22 rotates through various portions of its transfer path 34. The puck transfer mechanism 12 rotates about the puck transfer axis 32 at a relatively constant velocity VS but, as previously described, the servo motors 106 of the transfer mechanism 12 may be selectively operated to control the speed at which the pucks 22 (on their associated carriage unit 20) are moved/displaced circumferentially along the center plate, with a connecting arm 114 and drag link 120 transferring movement of the servo motor 106 to the carriage unit 20 on which puck 22 is mounted to cause an acceleration/deceleration of the puck 22 and circumferential displacement thereof along center plate 24. Thus, the description provided here below with regard to accelerating or decelerating a puck 22 is understood to refer to operation of a respective servo motor 106 that causes a corresponding change in velocity of the puck 22 (and carriage unit 20) operatively connected thereto. While an example puck velocity profile is described below, it is recognized that different puck velocity profiles could be implemented via selective control and operation of the servo motors 106.

When a puck 22 receives a continuous web material 186, the puck 22 may be moving at a substantially constant first velocity V1. A section of material, referred to hereafter as an insert or pad 188, is then cut from the continuous web 186. To create the pad 188, a first cut 190 is made proximate a leading puck edge 192 and a second cut 194 is made proximate the trailing puck edge 196. As previously described, cutting of the continuous web is performed via a positioning of respective anvils 56 at proper cutting positions relative to the pucks 22 and to the knife roll 48. The positioning of each anvil 56 (and anvil arm 52) between its two adjacent carriage units 20 is maintained relatively constant (i.e., mid-way therebetween) via its connection to the carriage units 20 by the scissor links 66. This controlled positioning of each anvil 56 ensures that the anvil 56 is in a proper position between its adjacent pucks 22 when it is time to cut the continuous web material 186, such as the first and second cuts 190, 194 indicated here.

Just after a pad 188 is cut from the web material 186, the puck 22 may be accelerated 198 and may be decelerated 200 thereafter back to a substantially constant velocity 202, which may be the first velocity V1. Sometime after the trailing edge cut 194 and prior to placement 204 of the pad 188 on a receiving surface 206, the puck 22 spins to a desired angle (via interaction of the spin cam follower 130 with the spin cam race 128) and the velocity of the puck 22 may change 208 (via operation of servo motor 106) to achieve a desirable predetermined circumferential spacing. Upon or after reaching a substantially constant 210 second velocity V2, the pad 188 is placed 204 on the receiving surface 206. After pad placement 204, the puck 22 is decelerated 212 to a substantially constant 214 first velocity V1 and is spun back to a web-receiving orientation. The process then begins anew.

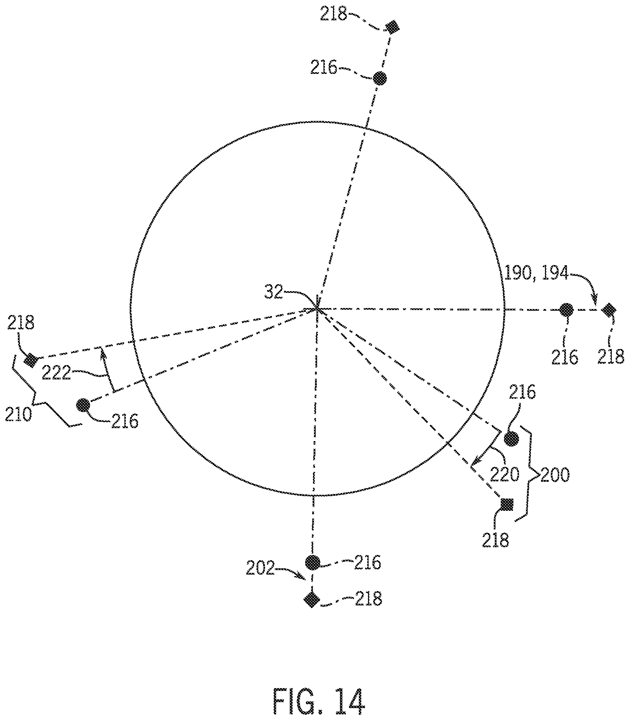

During periods of acceleration and deceleration, the pucks 22 change position relative to the major axis of rotation, the puck transfer axis 32 - i.e., the pucks may be circumferentially displaced. This can best be seen in FIG. 14. A first reference point 216 represents a point on the shaft 28 (FIG. 1) spinning about the puck transfer axis 32 at the relatively constant velocity VS during operation of the transfer mechanism 12. A second reference point 218 represents a position of a puck 22. While the shaft reference 216 may be rotating about the puck transfer axis 32 at a constant velocity, the position of the puck reference 218 with respect to the shaft 28 may change a desirable amount. Again, the position of the puck reference 218 with respect to the shaft 28 - and the increase/decrease in the degrees of rotation thereof responsive to acceleration/deceleration of the puck 22 - is controlled via selective operation of the servo motor associated with the puck 22.

As shown in FIG. 14, the shaft reference 216 is generally radially aligned with the puck reference 218 during times of cutting 190, 194. At the end 200 of the first acceleration, the puck reference 218 has changed position relative to the shaft reference 216 by a first distance 220. At the end 202 of the first deceleration period, the references 216, 218 are again aligned. Prior to pad placement 204, the puck 22 is again accelerated, and at the end 210 of the second acceleration the puck reference 218 has advanced beyond the shaft reference 216 by a second distance 222. The first distance 220 may be the same as, or different than, the second distance 222. Finally, at the end 214 of the second deceleration period, both references 216, 218 are aligned and ready for another revolution.

FIG. 15 shows a representative puck 22a in a first position P1. In the first position P1, the puck 22a receives a continuous web material 186 traveling in a first direction 224 at the first velocity. A vacuum is drawn through the carriage unit 20 (through the puck support 134 and the puck 22a) to support the web material 186 on the puck 22a surface. While receiving the web 186, the puck 22a is traveling about a puck transfer axis 32 in a second direction (i.e., machine direction 104), to which at this point P1 the first direction 224 is preferably substantially tangential. The puck 22a continues to move in the second direction 104 into a second position P2.

FIG. 16 depicts the puck 22a in the second position P2. In this position, the puck 22a is at the leading edge cut time 190 of FIG. 12. Here, a knife blade 58 of the knife roll 48 cooperates with a representative anvil 56a of the anvil wheel 46 to cut the web 186 proximate the leading edge 192 of the puck 22a. As previously described, the anvil 56a is moved to a proper cutting position between the leading and trailing pucks 22 adjacent thereto via its connection to the carriage units 20 of those pucks by the scissor links 66. After receipt of the web 186 and the cut made near the leading edge 192, the puck 22a proceeds to travel in the second direction 104 past the knife roll 48 to a third position P3.

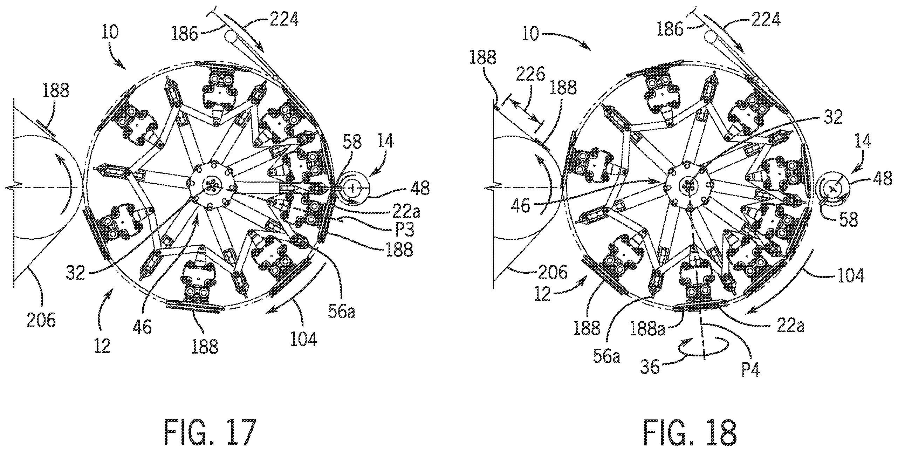

FIG. 17 shows the puck 22a in the third position P3. In this position P3, the puck 22a is at the trailing edge cut time 194 of FIG. 12. In this position P3, a knife blade 58 of knife roll 48 cooperates with an anvil 56 to cut the web 186 proximate the trailing edge 196 of the puck 22a to cut a pad 188a from the web 186. The pad 188a is held to the puck 22a by the vacuum, which was drawn previously. After the cut made near the trailing edge 196, the puck 22a proceeds to travel in the second direction 104 to a fourth position P4.

FIG. 18 shows the puck 22a in the fourth position P4. As mentioned previously, it is often desirable to spin the cut pad 188a to some predetermined angle prior to placement on a receiving surface 206. Here, the puck 22a is shown while in the midst of a spin. While FIG. 18 shows the puck 22a being spun in the fourth position P4, the puck 22a may spin to a desired angle any time after the trailing edge cut made at the third position P3 and before placement onto the receiving surface 206.

Besides rotation and spin of the pucks 22, the apparatus 10 may also change the circumferential spacing of the pucks 22. As previously described, the servo motor 106 associated with each puck 22 - and the individual control of each of the servo motors 106 - allows for altering of the circumferential spacing of the pucks 22 and the selective control of the pitch between each of the pucks 22, with the servo motors 106 enabling a large angular range of movement for each of the pucks 22. The displacement of the pucks 22 via the servo motors 106 thereby provides for a placement pitch of cut pads 188 that is different from the pitch at which the web material 186 was cut, with the ultimate circumferential spacing of the pucks 22 at the receiving surface 206 for placement of pads 188 thereon being a function of a desired placement pitch 226 and the speed at which the receiving surface 206 is traveling.

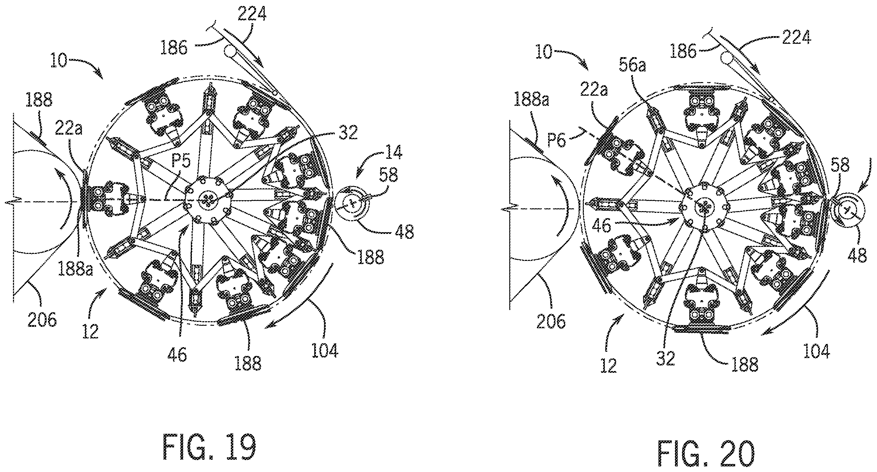

Upon achieving desired circumferential spacing, the puck 22a arrives in a fifth position P5. The puck 22a is shown in the fifth position P5 in FIG. 19. In this position P5, the puck 22a is at the middle of the placement time 204 shown in FIG. 12. The puck 22a has been situated at the correct placement pitch or distance 226 with respect to the puck 22 that preceded it. At this pitch or distance 226, the pad 188a is transferred to the receiving surface 206. At the time of placement, the vacuum that was drawn through the puck support 134 and puck 22a may be removed from at least a portion of the puck 22a, thereby allowing a smooth transfer of the cut insert 188a from the puck 22a to the receiving surface 206. After placing the pad 188a onto the receiving surface 206, the puck 22a continues in the second direction 104 to a sixth position P6.

FIG. 20 shows the puck 22a in the sixth position P6. The puck 22a is shown as having released the cut pad 188a onto the receiving surface 206. The puck 22a continues to move in the second direction 104 to a seventh position.

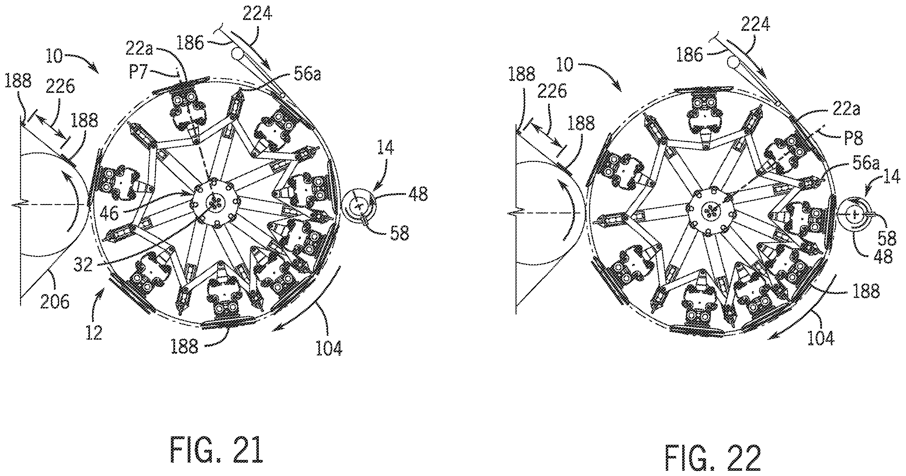

FIG. 21 depicts the seventh position P7 of the puck 22a. If the puck 22a and pad 188a were rotated after cutting to some predetermined angle prior to placement on the receiving surface 206, the puck 22a may need to be adjusted to a web-receiving orientation. While FIG. 21 shows the puck 22a spinning in the seventh position P7, the puck 22a may spin any time after the pad 188a has been placed on the receiving surface 206 and before the continuous web 186 is received.

It is recognized that the operation of the configurable cutting and transfer apparatus 10 shown and described above in FIGS. 12-22, including the described puck velocity profile, is for illustrative purposes only. That is, the rotation of the pucks 22 about the transfer path 34 may follow a different suitable velocity profile and/or spinning of the pucks 22 may vary from that described, according to additional embodiments of the invention, and that such embodiments are recognized as falling within the scope of the invention.

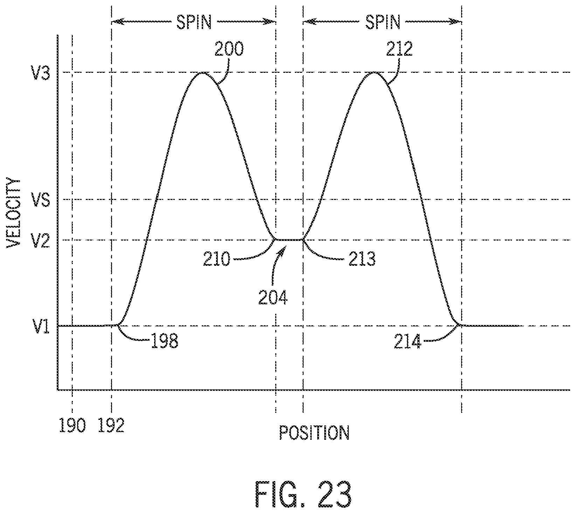

FIG. 23 illustrates one exemplary alternative puck velocity profile. The puck 22 moves at a substantially constant first velocity V1 during which time a first cut 190 is made proximate a leading puck edge 192 and a second cut 194 is made proximate the trailing puck edge 196. Following the second cut 194, the puck 22 is accelerated 198 to a third velocity V3 and decelerated 200 thereafter back to a substantially constant 210 second velocity V2 to achieve a desirable predetermined circumferential spacing between adjacent pucks 22. Sometime after the cut 194 and prior to placement 204 of the pad 188 on a receiving surface 206, the puck 22 spins to a desired angle (via interaction of the spin cam follower 130 with the spin cam race 128) and the velocity of the puck 22 may change 208 (via operation of servo motor 106). Upon or after reaching a substantially constant 210 second velocity V2, the pad 188 is placed 204 on the receiving surface 206. After pad placement 204, the puck 22 is accelerated 213 to the third velocity V3 and thereafter decelerated 212 to the substantially constant 214 first velocity V1 and is spun back to a web-receiving orientation. The process then begins anew.

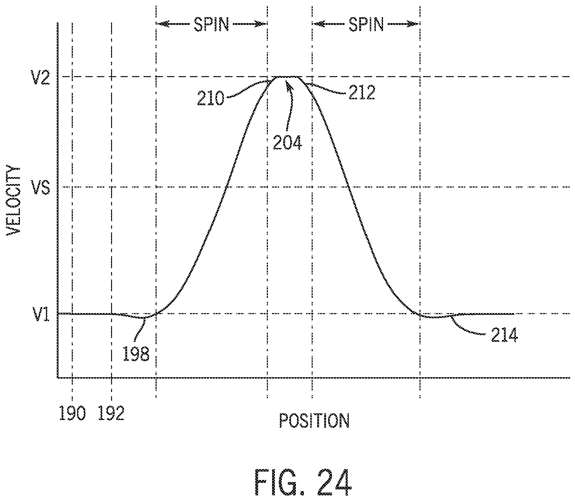

FIG. 24 illustrates another exemplary alternative puck velocity profile. The puck 22 moves at a substantially constant first velocity V1 during which time the first cut 190 is made and the second cut 194 is made. Following the second cut 194, the puck 22 is accelerated 198 to a substantially constant 210 second velocity V2 to achieve a desirable predetermined circumferential spacing between adjacent pucks 22. Sometime after the cut 194 and prior to placement 204 of the pad 188 on a receiving surface 206, the puck 22 spins to a desired angle (via interaction of the spin cam follower 130 with the spin cam race 128). At some point after reaching second velocity V2, the pad 188 is placed 204 on the receiving surface 206. After pad placement 204, the puck 22 is decelerated 212 to the substantially constant 214 first velocity V1 and is spun back to a web-receiving orientation. The process then begins anew.

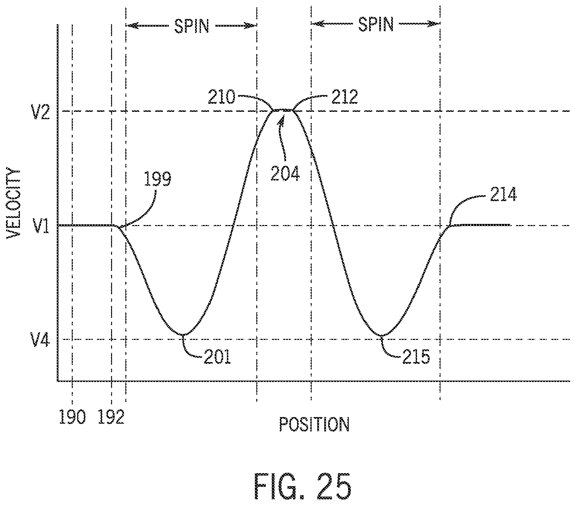

FIG. 25 illustrates yet another exemplary alternative puck velocity profile. The puck 22 moves at a substantially constant first velocity V1 during which time a first cut 190 is made proximate a leading puck edge 192 and a second cut 194 is made proximate the trailing puck edge 196. Following the second cut 194, the puck 22 is decelerated 199 to a fourth velocity V4 and accelerated 201 thereafter a substantially constant 210 second velocity V2 to achieve a desirable predetermined circumferential spacing between adjacent pucks 22. Sometime after the cut 194 and prior to placement 204 of the pad 188 on a receiving surface 206, the puck 22 spins to a desired angle (via interaction of the spin cam follower 130 with the spin cam race 128). Upon or after reaching a substantially constant 210 second velocity V2, the pad 188 is placed 204 on the receiving surface 206. After pad placement 204, the puck 22 is decelerated 212 to the fourth velocity V4 and thereafter accelerated 215 to the substantially constant 214 first velocity V1 and is spun back to a web-receiving orientation. The process then begins anew.

It is further recognized that aspects of the transfer mechanism 12 described above can be implemented in other systems or apparatuses, according to additional embodiments of the invention. Such systems or apparatuses may comprise a cutting and transfer apparatus of a different construction from the apparatus 10 previously described, where the transfer mechanism therein is identical to the transfer mechanism 12 but that includes a cutting system of a different configuration. Such systems or apparatuses may also comprise a pick-and-place system that is operable to receive (or "pick") a product or component part in one orientation, spin the component part to a desired predetermined angle, and then transfer (or "place") the component part onto another web or component for use in another step in a production process. Such a pick-and-place system would include a transfer mechanism identical to the transfer mechanism 12 previously described but would not include any type of cutting system 14 therein.

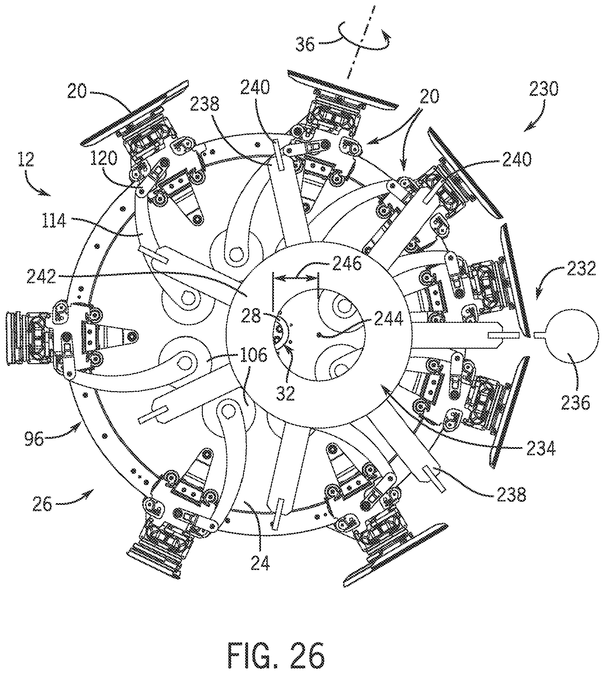

An example of a cutting and transfer apparatus 230 is provided in FIG. 26, according to an embodiment. As indicated, the apparatus 230 includes a transfer mechanism 12 as previously detailed, with the transfer mechanism 12 having a plurality of carriage units 20 and pucks 22 arranged about a center plate 24 to collectively form a puck wheel 26 of a desired configuration. The puck wheel 26 is coupled to a motor-driven shaft 28, which causes the puck wheel 26 - including the carriage units 20 and pucks 22 - to rotate about a puck transfer axis 32. To facilitate circumferential displacement of the carriage units 20 and pucks 22 along pitch rails 96 provided about a circumference of the center plate 24, the transfer mechanism 12 includes a plurality of servo motors 106. A dedicated servo motor 106 is provided for each carriage unit 20 to enable displacement of its carriage unit 20 (and puck 22) along the pitch rails 96. In operation, a servo motor 106 may rotate both clockwise and counterclockwise, such that a connecting arm 114 coupling the servo motor 106 to the carriage unit 20 (via a drag link 120) moves back and forth in a reciprocating, windshield wiper type pattern. Via this motion of the connecting arm 114, and via the drag link 120 coupling the connecting arm 114 to the carriage unit 20, rotational motion of a servo motor 106 may therefore be transformed into a circumferential displacement of a respective carriage unit 20 along the pitch rails 96. In addition to such circumferential displacement of the carriage units 20 and pucks 22, each puck may be radially spun about a puck spin axis 36 as previously described (i.e., via interaction of a spin cam follower 130 on the carriage unit 20 with a spin cam race 128 provided on a barrel cam 128 situated about the transfer axis 32, as shown in FIG. 1).

The apparatus 230 also includes a cutting system 232 that comprises an anvil wheel 234 and a knife roll 236 that interact with one another to cut discrete pads or inserts from a continuous web that is provided to the cutting and transfer apparatus 10. The anvil wheel 234 is sized smaller than the transfer mechanism 12 (i.e., a diameter of the anvil wheel 234 is less than the diameter of the transfer mechanism), such that the anvil wheel 234 is generally positioned within the circumference of the transfer mechanism 12. The anvil wheel 234 includes a plurality of anvil arms 238 (each with an anvil 240 provided thereon) that extend radially outward from a central hub 242, with the anvil arms 238 arranged about the hub in a fixed position. The hub 242 is coupled to a drive shaft (not shown) that is aligned along an anvil wheel axis 244, with the anvil wheel axis 244 offset from the puck transfer axis 32. In the illustrated embodiment, the anvil wheel 234 has fewer anvils 240 than the number of pucks 22 provided on the transfer mechanism 12, which allows a greater offset 246 between the anvil wheel axis 244 and the puck transfer axis 32. The eccentric offset causes a virtual withdrawal of the anvils 240 from between adjacent pucks 22 when the anvils 240 are not at a cutting position where they are desired to interact with the knife roll 236 to cut the continuous web.

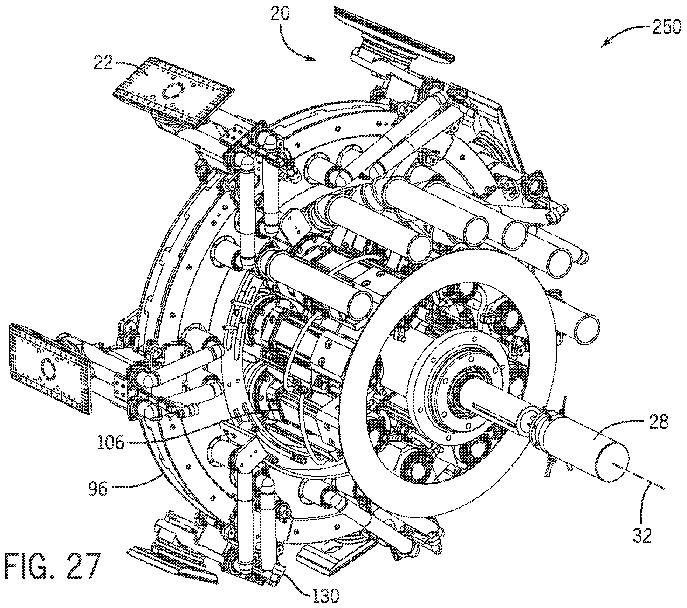

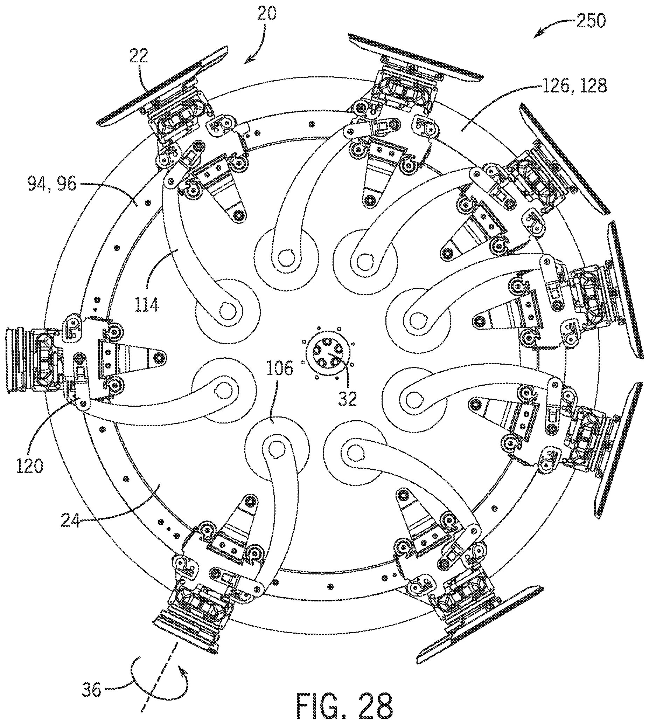

An example of a pick-and-place system is provided in FIGS. 27 and 28, according to an embodiment. As previously indicated, the pick-and-place system 250 (hereafter "system") is, in general, equivalent in construction and operation to the transfer mechanism 12 previously described, but with the cutting system 14 removed. That is, in operation of the system 250, discrete items (e.g., discrete inserts, pads, sections of web material, etc.) that were previously formed in earlier step(s) of a production process are provided directly to the pucks 22 for pick-up and transfer thereby, with no cutting of an incoming continuous web being performed. The system 250 thus includes a plurality of carriage units 20 and pucks 22 arranged about a center plate 24 to collectively form a puck wheel 26 of a desired configuration. The puck wheel 26 is coupled to a motor-driven shaft 28, which causes the puck wheel 26 - including the carriage units 20 and pucks 22 - to rotate about a puck transfer axis 32. To facilitate circumferential displacement of the carriage units 20 and pucks 22 along the pitch rails 96 provided about a circumference of the center plate 24, the transfer mechanism 12 includes a plurality of servo motors 106. A dedicated servo motor 106 is provided for each carriage unit 20 to enable displacement of its carriage unit 20 (and puck 22) along the pitch rails 96. In operation, a servo motor 106 may rotate both clockwise and counterclockwise, such that a connecting arm 114 coupling the servo motor 106 to the carriage unit 20 (via a drag link 120) moves back and forth in a windshield wiper type pattern. Via this motion of the connecting arm 114, and via the drag link 120 coupling the connecting arm 114 to the carriage unit 20, rotational motion of a servo motor 106 may therefore be transformed into a circumferential displacement of a respective carriage unit 20 along the pitch rails 96. In addition to such circumferential displacement of the carriage units 20 and pucks 22, each puck may be radially spun about a puck spin axis 36 via interaction of a spin cam follower 130 on the carriage unit 20 with a spin cam race 128 provided on a barrel cam 126 situated about the transfer axis 32 and positioned on the drive side 30 of the center plate 24.

Beneficially, embodiments of the invention thus provide discrete article transfer apparatuses and cutting and transfer apparatus that enable adjustments in operation thereof for accommodating transferring (and cutting) of articles of differing types and sizes. The apparatuses are able to accommodate the manufacture of a large range of product sizes due to the speed capabilities and circumferential puck displacement range provided by the transfer mechanism. That is, the use of servo motors in the transfer mechanism for driving the individual carriage units allows for greater angular displacement and pitch spacing between the pucks, so as to accommodate larger chassis sizes (and puck sizes) on the transfer mechanism. For cutting and transfer apparatuses, the structure of the cutting system - with the anvil wheel and knife roll being translatable relative to the transfer mechanism - allows for the cutting system to similarly accommodate the manufacture of a large range of product sizes. A repositioning of the anvil wheel and knife roll can be performed in association with any changes to the size and/or operating velocity of the transfer mechanism without having to swap out components of the cutting system. Camming of the knife roll is also provided via the use of a separate servo motor, such that operation of the knife roll can be synched with changes to the transfer mechanism operating parameters. Most or all of the reconfiguring of the cutting and transfer apparatus may be achieved via a push-button changeover, with minimal manual reconfiguring of the apparatus being required, so as to minimize apparatus downtime when performing size change adjustments.

Further benefits are provided by the structure of the cutting system and the mechanical coupling thereof to the transfer mechanism. That is, the anvil wheel is structured such that each anvil arm is pivotable relative to a central anvil hub, while each anvil arm is also coupled to the chassis of the two carriage units between which the anvil arm is positioned. Each anvil arm is coupled to its adjacent carriage unit chassis via scissor links extending therebetween, with the scissor links being pivotably connected to the carriage unit chassis and the anvil arm. The coupling of each anvil arm to its two adjacent carriage units via the scissor links keeps the anvil arm centered between the carriage units, with the circumferential displacement of the carriage units via their respective servo motors thus dictating the positioning of the anvil arms. The anvil arms are thus prevented from colliding or otherwise coming into contact with the carriage units (pucks) regardless of the specific configuration of the transfer mechanism for a particular size change operation and/or in the event a servo motor malfunctioning, thereby providing an operational safeguard in the apparatus.

Therefore, according to one embodiment of the invention, a cutting and transfer apparatus includes a cutting system configured to cut an incoming continuous web of material into a plurality of discrete articles and a transfer mechanism operable with the cutting system to transfer and rotate the plurality of discrete articles from at least a web receiving location to an article placement location. The transfer mechanism further includes a drive shaft rotatable about a transfer axis and a puck wheel mounted to the drive shaft to rotate therewith about the transfer axis. The puck wheel includes a plurality of carriage units that rotate about the transfer axis to travel along a transfer path about the transfer axis from at least the web receiving location to the article placement location, each of the plurality of carriage units including a puck that is selectively operable to carry a discrete article thereon and reorient the article as the puck travels between the web receiving location and the article placement location. The puck wheel also includes a plurality of servo motors each operably connected to a respective carriage unit of the plurality of carriage units via a connecting arm, and with each of the plurality of servo motors operable to alter positioning of its respective carriage unit along at least a portion of the transfer path.

According to another embodiment of the invention, a pick-and-place system for transferring and rotating a plurality of discrete articles from at least an article receiving location to an article placement location is disclosed. The pick-and-place system includes a drive shaft rotatable about a transfer axis, a center plate mounted to the drive shaft to rotate therewith about the transfer axis, and a plurality of carriage units positioned about the center plate to rotate therewith to travel along a transfer path about the transfer axis from at least the article receiving location to the article placement location, with each of the plurality of carriage units including a puck that is selectively operable to carry a discrete article thereon and reorient the article as the puck travels between the article receiving location and the article placement location. The pick-and-place system also includes a plurality of servo motors mounted to the center plate, with each servo motor operably connected to a respective carriage unit of the plurality of carriage units via a connecting arm, and with each of the plurality of servo motors operable to alter positioning of its respective carriage unit with respect to the center plate along at least a portion of the transfer path.

According to yet another embodiment of the invention, a cutting and transfer apparatus includes a cutting system configured to cut an incoming web of material into a plurality of discrete articles and a transfer mechanism operable with the cutting system to transfer and rotate the plurality of discrete articles from at least a web receiving location to an article placement location. The transfer mechanism further includes a drive shaft rotatable about a transfer axis, a center plate mounted to the drive shaft to rotate therewith about the transfer axis, and a plurality of carriage units positioned about the center plate to rotate therewith to travel along a transfer path about the transfer axis from at least the web receiving location to the article placement location, with each of the plurality of carriage units including a puck that is selectively operable to carry a discrete article thereon and reorient the article as the puck travels between the web receiving location and the article placement location. The cutting system includes an anvil wheel having an anvil hub coupled to and driven by an anvil shaft to rotate about an anvil wheel axis and a plurality of anvil arms pivotably connected to and extending radially outward from the anvil hub, each of the plurality of anvil arms having thereon an anvil that cooperates with a knife roll of the cutting system to cut the incoming web of material. The plurality of anvil arms is equal in number to the plurality of carriage units, with the plurality of anvil arms interspersed with the plurality of carriage units such that each anvil arm is positioned between a pair of adjacent carriage units. Each anvil arm is mechanically coupled to the pair of adjacent carriage units between which it is positioned to control positioning of the anvil relative to the pair of adjacent carriage units.

According to still another embodiment of the invention, a method for configuring a cutting and transfer apparatus includes providing a cutter mechanism configured to cut an incoming web of material into a plurality of discrete articles and providing a transfer mechanism operable with the cutter mechanism to transfer and rotate the plurality of discrete articles from at least a web receiving location to an article placement location. In providing the transfer mechanism, the method further includes providing a drive shaft having a mounting structure coupled thereto, the drive shaft and mounting structure rotatable about a transfer axis and mounting a plurality of carriage units to the mounting structure so that the plurality of carriage units are rotatable with the mounting structure to travel along a transfer path about the transfer axis from at least the web receiving location to the pad placement location, with each of the plurality of carriage units including a puck that is selectively operable to carry a discrete article thereon and reorient the article as the puck travels between the web receiving location and the article placement location. The method also includes operably connecting a servo motor to each of the plurality of carriage units via a connecting arm, with each servo motor selectively operable to alter positioning of its respective carriage unit with respect to the mounting structure by circumferentially displacing the carriage unit along the transfer path.

According to still another embodiment of the invention, a cutting and transfer apparatus includes a cutting system configured to cut an incoming web of material into a plurality of discrete articles and a transfer mechanism operable with the cutting system to transfer and rotate the plurality of discrete articles from at least a web receiving location to an article placement location. The transfer mechanism further includes a drive shaft rotatable about a transfer axis, a mounting structure rotatable about the transfer axis, a plurality of carriage units coupled to the mounting structure and configured to travel along a transfer path about the transfer axis from at least the web receiving location to the article placement location, and a plurality of pucks coupled to the plurality of carriage units to carry discrete articles thereon and reorient the articles as the plurality of pucks travel between the web receiving location and the article placement location. The cutting system includes an anvil wheel having an anvil hub coupled to and driven by an anvil shaft to rotate about an anvil wheel axis and a plurality of anvil arms pivotably connected to and extending radially outward from the anvil hub, each of the plurality of anvil arms having thereon an anvil that cooperates with a knife roll of the cutting system to cut the incoming web of material. The plurality of anvil arms is equal in number to the plurality of carriage units, with the plurality of anvil arms interspersed with the plurality of carriage units such that each anvil arm is positioned between a pair of adjacent carriage units. Each anvil arm is mechanically coupled to the pair of adjacent carriage units between which it is positioned to control positioning of the anvil relative to the pair of adjacent carriage units.

## Claims

1. A cutting and transfer apparatus (10; 230) comprising:
a cutting system (14; 232) configured to cut an incoming continuous web of material into a plurality of discrete articles; and
a transfer mechanism (12) operable with the cutting system (14; 232) to transfer and rotate the plurality of discrete articles from at least a web receiving location to an article placement location, the transfer mechanism (12) comprising:
a drive shaft (28) rotatable about a transfer axis (32);
a puck wheel (26) mounted to the drive shaft (28) to rotate therewith about the transfer axis (32), the puck wheel (26) including:
a plurality of carriage units (20) that rotate about the transfer axis (32) to travel along a transfer path (34) about the transfer axis (32) from at least the web receiving location to the article placement location, each of the plurality of carriage units (20) including a puck (22) that is selectively operable to carry a discrete article thereon and reorient the article as the puck (22) travels between the web receiving location and the article placement location; and
a plurality of servo motors (106) each operably connected to a respective carriage unit of the plurality of carriage units (20) via a connecting arm (114), and with each of the plurality of servo motors (106) operable to alter positioning of its respective carriage unit along at least a portion of the transfer path (34).

2. The apparatus (10) of claim 1 wherein the puck wheel (26) comprises a center plate (24) mounted to the drive shaft (28) to rotate therewith about the transfer axis (32), with the plurality of carriage units (20) mounted to and positioned about the center plate (24) and with the plurality of servo motors (106) mounted to the center plate (24); and wherein each of the plurality of servo motors (106) is operable to alter positioning of its respective carriage unit with respect to the center plate (24) along at least the portion of the transfer path (34).

3. The apparatus (10) of claim 2 further comprising an annular rail structure (94) positioned about an outer circumference of the center plate (24), the rail structure (94) including pitch rails (96) on which the plurality of carriage units (20) is mounted to be movable therealong.

4. The apparatus (10) of claim 3 wherein each of the plurality of carriage units (20) comprises:
a chassis (132) including a pair of frame members (136,138) positioned on opposing sides of the rail structure (94);
a plurality of rollers (140,142) coupled to the chassis (132) and in rolling engagement with an inner edge (100) and outer edge (102) of the pitch rails (96) to secure the carriage unit to the rail structure (94) and provide for translation of the carriage unit along the pitch rails (96); and
a puck support (134) positioned on the chassis (132) and movable relative thereto via a mating of rail guides of the puck support (134) with the pitch rails (96), the puck support (134) oriented generally orthogonal to the pitch rails (96) and comprising a puck mount (144) on one end thereof that is configured to receive the puck (22).

5. The apparatus (10) of claim 4 wherein the transfer mechanism (12) comprises:
a stationary vacuum manifold (42) positioned adjacent the center plate (24) and configured to transfer a vacuum from a vacuum source to an interior volume of the center plate (24); and
a plurality of vacuum tubes coupled to the center plate (24), with one or more vacuum tubes of the plurality of vacuum tubes fluidly connecting the interior volume of the center plate (24) with a respective carriage unit of the plurality of carriage units (20) to transfer the vacuum thereto,
wherein, optionally, the plurality of vacuum tubes comprise telescoping vacuum tubes (44) each rotatably coupled to the center plate (24) to swivel relative thereto, and wherein each telescoping vacuum tube (44) is collapsible and extendible to decrease and increase a length thereof, such that a length of the telescoping vacuum tube (44) can change to accommodate movement of a respective carriage unit along the rail structure (94).

6. The apparatus (10; 230) of claim 1 wherein the cutting system (14; 232) comprises:
a knife roll (48; 236) including one or more knives (58) thereon; and
an anvil wheel (46; 234) comprising:
an anvil hub (50; 242) coupled to and driven by an anvil shaft (76) to rotate about an anvil wheel axis (81; 244);
a plurality of anvil arms (52; 238) pivotably connected to and extending radially outward from the anvil hub (50; 242); and
an anvil (56; 240) positioned at an end of each of the plurality of anvil arms (52; 238), with the anvils (56) periodically cooperating with the one or more knives (58) of the knife roll (48; 236) to cut the incoming web of material; and
a linkage system (64) that mechanically couples the plurality of anvil arms (52; 238) together.

7. The apparatus (10) of claim 6 wherein the linkage system (64) comprises a pair of scissor links (66) provided between each adjacent pair of anvil arms of the plurality of anvil arms (52; 238), with a first link of the pair of scissor links (66) rotatably coupled to a first anvil arm of the adjacent pair of anvil arms (52; 238) and a second link of the pair of scissor links (66) rotatably coupled to a second anvil arm of the adjacent pair of anvil arms (52; 238).

8. The apparatus (10) of claim 7 wherein each of the plurality of anvil arms (52; 238) comprises a linear slide (71) by which respective links of the linkage system (64) are coupled thereto, the linear slide (71) providing a radially inward and outward movement of a pivot point (70) at which the links are coupled to the anvil arms (52; 238).

9. The apparatus of claim 6 wherein the cutting system (14; 232) further comprises:
a belt drive (80) that drives the anvil shaft (76) to cause rotation of the anvil wheel (46; 234); and
a knife roll servo motor (82) coupled to the knife roll (48; 236) to drive rotation of the knife roll (48; 236);
wherein the knife roll servo motor (82) is separate from the belt drive (80), such that the knife roll (48; 236) is driven separately from the anvil wheel (46; 234) via camming of the knife roll servo motor (82),
and, optionally, further comprises:
a lower cutter box assembly (86) having tracks (88) formed along a top surface thereof;
an upper cutter box assembly (84) positioned above the lower cutter box assembly (86) and movable laterally relative to the lower cutter box assembly (86) along the tracks (88); and
an adjustment mechanism actuatable to cause lateral movement of the upper cutter box assembly (84) along the tracks (88);
wherein each of the anvil wheel (46; 234) and the knife roll (48; 236) are mounted on the upper cutter box assembly (84), such that lateral movement of the upper cutter box assembly (84) causes the anvil wheel (46; 234) and the knife roll (48; 236) to move laterally relative to the transfer mechanism.

10. The apparatus (10) of claim 6 further comprising a human-machine interface (HMI) (18) configured to:
receive operator inputs directed to a reconfiguring of the transfer mechanism (12) and/or the cutting system (14; 232); and
generate commands responsive to the operator inputs that cause one or more operational settings of the transfer mechanism (12) and/or the cutting system (14; 232) to be modified;
wherein the one or more operational settings comprise at least one of a rotational speed of the drive shaft (28), a pitch between adjacent pucks (22), a speed of the plurality of servo motors (106), a lateral positioning of the anvil wheel (46; 234) and the knife roll (48; 236), a rotational speed of the anvil shaft (76), and a speed of the knife roll servo motor (82),
and, optionally, further comprising a slip ring (124) positioned on the drive shaft (28), and wherein communications and/or power connections to the plurality of servo motors (106) are routed through the slip ring (124).

11. The apparatus (10) of claim 1 wherein the connecting arm (114) comprises a curved, arcuate-shaped connecting arm.

12. A pick-and-place system (250) for transferring and rotating a plurality of discrete articles from at least an article receiving location to an article placement location, the pick-and-place system comprising:
a drive shaft (28) rotatable about a transfer axis (32);
a center plate (24) mounted to the drive shaft (28) to rotate therewith about the transfer axis (32);
a plurality of carriage units (20) positioned about the center plate (24) to rotate therewith to travel along a transfer path about the transfer axis (32) from at least the article receiving location to the article placement location, each of the plurality of carriage units (20) including a puck (22) that is selectively operable to carry a discrete article thereon and reorient the article as the puck (22) travels between the article receiving location and the article placement location; and
a plurality of servo motors (106) mounted to the center plate (24), with each servo motor operably connected to a respective carriage unit of the plurality of carriage units (20) via a connecting arm (114), and with each of the plurality of servo motors (106) operable to alter positioning of its respective carriage unit with respect to the center plate (24) along at least a portion of the transfer path.

13. The pick-and-place system (250) of claim 12 wherein the center plate (24) comprises an annular rail structure positioned about an outer circumference thereof, the rail structure including pitch rails (96) on which the plurality of carriage units (20) is mounted to be movable therealong.

14. The pick-and-place system (250) of claim 13 wherein each of the plurality of carriage units (20) comprises:
a chassis including a pair of frame members, with the frame members positioned on opposing sides of the rail structure;
a plurality of rollers coupled to the chassis and in rolling engagement with an inner edge and outer edge of the pitch rails (96) to secure the carriage unit to the rail structure and provide for translation of the carriage unit along the pitch rails (96); and
a puck support positioned on the chassis and movable relative thereto via a mating of rail guides of the puck support with the pitch rails (96), the puck support oriented generally orthogonal to the pitch rails (96) and comprising a puck mount on one end thereof that is configured to receive the puck (22).

15. The pick-and-place system of claim 13 comprising:
a stationary vacuum manifold positioned adjacent the center plate (24) and configured to transfer a vacuum from a vacuum source to an interior volume of the center plate (24); and
a plurality of vacuum tubes coupled to the center plate (24), with one or more vacuum tubes of the plurality of vacuum tubes fluidly connecting the interior volume of the center plate (24) with a respective carriage unit of the plurality of carriage units (20) to transfer the vacuum thereto;
wherein each vacuum tube is rotatably coupled to the center plate (24) to swivel relative thereto, and wherein a length and/or configuration of each vacuum tube is variable to accommodate movement of a respective carriage unit along the rail structure.

16. The pick-and-place system of claim 13 further comprising:
a plurality of motor drives secured on the center plate (24) and operably connected to the plurality of servo motors (106) to control operation thereof; and
a human-machine interface (HMI) in communication with the plurality of motor drives to provide commands thereto, the HMI configured to:
receive operator inputs directed to a reconfiguring of the pick-and-place system; and
provide commands to the plurality of motor drives to cam the plurality of servo motors (106) according to a pre-determined velocity profile associated with the operator inputs and thereby alter positioning of the plurality of carriage units (20) with respect to the center plate (24) along the at least the portion of the transfer path.

17. A method for configuring a cutting and transfer apparatus (10), the method comprising:
providing a cutter mechanism (14) configured to cut an incoming web of material into a plurality of discrete articles; and
providing a transfer mechanism (12) operable with the cutter mechanism (14) to transfer and rotate the plurality of discrete articles from at least a web receiving location to an article placement location, wherein providing the transfer mechanism (12) comprises:
providing a drive shaft (28) having a mounting structure coupled thereto, the drive shaft (28) and mounting structure rotatable about a transfer axis (32); and
mounting a plurality of carriage units (20) to the mounting structure so that the plurality of carriage units (20) are rotatable with the mounting structure to travel along a transfer path (34) about the transfer axis (32) from at least the web receiving location to the pad placement location, with each of the plurality of carriage units (20) including a puck (22) that is selectively operable to carry a discrete article thereon and reorient the article as the puck (22) travels between the web receiving location and the article placement location; and
operably connecting a servo motor (106) to each of the plurality of carriage units (22) via a connecting arm (114), with each servo motor selectively operable to alter positioning of its respective carriage unit with respect to the mounting structure by circumferentially displacing the carriage unit along the transfer path (34).

18. The method of claim 17 wherein the mounting structure comprises a center plate (24) and, wherein mounting the plurality of carriage units (20) comprises mounting the plurality of carriage units (20) onto an annular rail structure (94) positioned about an outer circumference of the center plate (24), the rail structure (94) including pitch rails (96) on which the plurality of carriage units (20) is mounted to be movable therealong.

19. The method of claim 17 further comprising coupling a respective puck (22) to each of the plurality of carriage units (20) to provide pucks (22) that accommodate a desired article size that is to be produced by the cutting and transfer apparatus (10).

20. The method of claim 18 further comprising:
providing inputs to the cutting and transfer apparatus (10) from a human-machine interface (HMI) (18) indicative of a product size to be produced by the cutting and transfer apparatus (10); and
positioning the cutter mechanism (14) relative to the transfer mechanism (12) based on the inputs from the HMI (18), wherein positioning the cutter mechanism (14) comprises positioning an anvil wheel (46; 234) and a knife roll (48; 236) of the cutter mechanism (14) relative to the transfer mechanism (12) based on the inputs from the HMI (18) to cut the incoming web of material at a cutting location.
and, optionally, further comprising:
controlling a rotational speed of the drive shaft (28) and the anvil wheel (46; 234) based on the inputs from the HMI (18), the rotational speed of the drive shaft (28) and the rotational speed of the anvil wheel (46; 234) being kept equal;
controlling a rotational speed of the servo motors (106) based on the inputs from the HMI (18); and
controlling a rotational speed of the knife roll (48; 236), via camming of a knife roll servo motor (82) operatively connected to the knife roll (48; 236), based on the inputs from the HMI (18).

21. A method for initiating product changeover in a transfer apparatus (10), the transfer apparatus (10) including a puck wheel (26) and an anvil wheel (46; 234) driven by at least one drive shaft (28), a plurality of carriage units (20) operatively coupled along a circumference of the puck wheel (26), each carriage unit including an interchangeable puck (22), the anvil wheel (46; 234) having a plurality of anvil arms (52; 238) pivotably connected to an anvil hub (50; 242) and extending radially outward from the anvil hub (50; 242), and a rotating blade in cooperation with the anvil arms (52; 238), the method comprising:
optionally removing each puck (22) having a first size from its corresponding carriage unit;
optionally attaching a puck (22) having a second size to each corresponding carriage unit;
sending commands, sequentially or simultaneously, to one or more controllers that control a drive shaft motor, a plurality of carriage unit motors, and a blade motor, to:
modify a rotational speed of the drive shaft (26) to modify the velocity of the puck wheel (26) and anvil wheel (46; 234);
accelerate a selected carriage unit to linearly displace the selected carriage unit from a first position to a second position along a portion of the circumference of the puck wheel (26), wherein displacement of the selected carriage unit changes an angular position of corresponding anvil arms (52; 238) adjacent the selected carriage unit;
adjust a rotational speed and angular position of the rotating blade so that the rotating blade meets a distal end of a corresponding anvil arm (52; 238), and cuts a continuous web of material disposed between the rotating blade and the distal end of the corresponding anvil arm (52; 238); and
deaccelerate a selected carriage unit to linearly displace the selected carriage unit from the second position to the first position.

22. The method of claim 21, wherein the step of optionally removing each puck (22) of the first size and attaching each puck (22) of the second size is performed by a robotic mechanism or by a human.

23. The method of claim 21, including providing each carriage unit with a quick connector engagement structure or fastenerless attachment to facilitate efficient swapping out of each puck (22) on its corresponding carriage unit.

24. The method of claim 21, further including providing a servo motor (106) to control the acceleration of each carriage unit.

25. The method of claim 21, further including providing a servo motor (82) control a rotational speed and angular position of the rotating blade so that the rotating blade meets a distal end of a corresponding anvil arm (52; 238), and cuts the continuous web of material disposed between the rotating blade and the distal end of the corresponding anvil arm (52; 238).

## Patentansprüche

1. Schneid- und Übertragungsvorrichtung (10; 230), umfassend:
ein Schneidsystem (14; 232), das so konfiguriert ist, dass es eine eingehende kontinuierliche Materialbahn in eine Vielzahl einzelner Artikel schneidet; und
einen Übertragungsmechanismus (12), der mit dem Schneidsystem (14; 232) betreibbar ist, um die Vielzahl einzelner Artikel von mindestens einer Bahnaufnahmestelle zu einer Artikelplatzierungsstelle zu übertragen und zu drehen, wobei der Übertragungsmechanismus (12) Folgendes umfasst:
eine Antriebswelle (28), die um eine Übertragungsachse (32) drehbar ist;
ein Puckrad (26), das an der Antriebswelle (28) montiert ist, um sich mit dieser um die Übertragungsachse (32) zu drehen, wobei das Puckrad (26) Folgendes umfasst:
eine Vielzahl von Wageneinheiten (20), die sich um die Übertragungsachse (32) drehen, um entlang eines Übertragungswegs (34) um die Übertragungsachse (32) von mindestens der Bahnaufnahmestelle zur Artikelplatzierungsstelle zu fahren, wobei jede der Vielzahl von Wageneinheiten (20) einen Puck (22) enthält, der selektiv betätigt werden kann, um einen einzelnen Artikel darauf zu tragen und den Artikel neu auszurichten, während sich der Puck (22) zwischen der Bahnaufnahmestelle und der Artikelplatzierungsstelle bewegt; und
eine Vielzahl von Servomotoren (106), die jeweils über einen Verbindungsarm (114) betriebsmäßig mit einer jeweiligen Wageneinheit der Vielzahl von Wageneinheiten (20) verbunden sind, und wobei jeder der Vielzahl von Servomotoren (106) betreibbar ist, um die Positionierung seiner jeweiligen Wageneinheit entlang mindestens eines Abschnitts des Übertragungswegs (34) zu ändern.

2. Vorrichtung (10) nach Anspruch 1, wobei das Puckrad (26) eine Mittelplatte (24) umfasst, die an der Antriebswelle (28) montiert ist, um sich mit dieser um die Übertragungsachse (32) zu drehen, wobei die Vielzahl von Wageneinheiten (20) an der Mittelplatte (24) montiert und um diese herum positioniert sind und wobei die Vielzahl von Servomotoren (106) an der Mittelplatte (24) montiert sind; und wobei jeder der Vielzahl von Servomotoren (106) betreibbar ist, um die Positionierung seiner jeweiligen Wageneinheit in Bezug auf die Mittelplatte (24) entlang zumindest des Abschnitts des Übertragungswegs (34) zu ändern.

3. Vorrichtung (10) nach Anspruch 2, weiterhin umfassend eine ringförmige Schienenstruktur (94), die um einen Außenumfang der Mittelplatte (24) positioniert ist, wobei die Schienenstruktur (94) Neigungsschienen (96) umfasst, auf denen die Vielzahl von Wageneinheiten (20) montiert ist, um entlang dieser beweglich zu sein.

4. Vorrichtung (10) nach Anspruch 3, wobei jede der Vielzahl von Wageneinheiten (20) Folgendes umfasst:
ein Fahrgestell (132), das ein Paar Rahmenelemente (136,138) enthält, die auf gegenüberliegenden Seiten der Schienenstruktur (94) positioniert sind;
eine Vielzahl von Rollen (140,142), die mit dem Fahrgestell (132) verbunden sind und in Rolleingriff mit einer Innenkante (100) und einer Außenkante (102) der Neigungsschienen (96) stehen, um die Wageneinheit an der Schienenkonstruktion (94) zu befestigen und um eine Verschiebung der Wageneinheit entlang der Neigungsschienen (96) zu ermöglichen; und
eine Puckhalterung (134), die auf dem Fahrgestell (132) positioniert und relativ zu diesem über eine Passung von Schienenführungen der Puckhalterung (134) mit den Neigungsschienen (96) beweglich ist, wobei die Puckhalterung (134) im Allgemeinen orthogonal zu den Neigungsschienen (96) ausgerichtet ist und an einem Ende davon eine Puckhalterung (144) aufweist, die zur Aufnahme des Pucks (22) konfiguriert ist.

5. Vorrichtung (10) nach Anspruch 4, wobei der Übertragungsmechanismus (12) Folgendes umfasst:
einen stationären Vakuumverteiler (42), der neben der Mittelplatte (24) positioniert und so konfiguriert ist, dass er ein Vakuum von einer Vakuumquelle an ein Innenvolumen der Mittelplatte (24) überträgt; und
eine Vielzahl von Vakuumröhren, die mit der Mittelplatte (24) gekoppelt sind, wobei eine oder mehrere Vakuumröhren der Vielzahl von Vakuumröhren das Innenvolumen der Mittelplatte (24) mit einer jeweiligen Wageneinheit der Vielzahl von Wageneinheiten (20) strömungstechnisch verbinden, um das Vakuum darauf zu übertragen,
wobei optional die Vielzahl von Vakuumröhren teleskopierbare Vakuumröhren (44) umfassen, die jeweils drehbar mit der Mittelplatte (24) gekoppelt sind, um relativ zu dieser zu schwenken, und wobei jede teleskopierbare Vakuumröhre (44) zusammenklappbar und ausziehbar ist, um ihre Länge zu verkürzen und zu vergrößern, so dass sich eine Länge der teleskopierbaren Vakuumröhre (44) ändern kann, um der Bewegung einer jeweiligen Wageneinheit entlang der Schienenstruktur (94) Rechnung zu tragen.

6. Vorrichtung (10; 230) nach Anspruch 1, wobei das Schneidsystem (14; 232) Folgendes umfasst:
eine Messerwalze (48; 236) mit einem oder mehreren Messern (58) darauf; und
ein Ambossrad (46; 234), umfassend:
eine Ambossnabe (50; 242), die mit einer Ambosswelle (76) gekoppelt ist und von dieser angetrieben wird, um sich um eine Ambossradachse (81; 244) zu drehen;
eine Vielzahl von Ambossarmen (52; 238), die schwenkbar mit der Ambossnabe (50; 242) verbunden sind und sich von dieser radial nach außen erstrecken; und
einen Amboss (56; 240), der an einem Ende jedes der Vielzahl von Ambossarmen (52; 238) positioniert ist, wobei die Ambosse (56) periodisch mit einem oder mehreren Messern (58) der Messerwalze (48; 236) zusammenwirken, um die ankommende Materialbahn zu schneiden; und
ein Verbindungssystem (64), das die Vielzahl von Ambossarmen (52; 238) mechanisch miteinander koppelt.

7. Vorrichtung (10) nach Anspruch 6, wobei das Verbindungssystem (64) ein Paar Scherenverbindungen (66) umfasst, die zwischen jedem benachbarten Paar Ambossarme der Vielzahl von Ambossarmen (52; 238) vorgesehen sind, wobei ein erstes Glied des Paars Scherenglieder (66) drehbar mit einem ersten Ambossarm des benachbarten Paares Ambossarme (52; 238) gekoppelt ist und ein zweites Glied des Paars Scherenglieder (66) drehbar mit einem zweiten Ambossarm des benachbarten Paars Ambossarme (52; 238) gekoppelt ist.

8. Vorrichtung (10) nach Anspruch 7, wobei jeder der Vielzahl von Ambossarmen (52; 238) einen linearen Schlitten (71) umfasst, durch den entsprechende Verbindungen des Verbindungssystems (64) damit gekoppelt sind, wobei der lineare Schlitten (71) eine radiale Einwärts- und Auswärtsbewegung eines Drehpunkts (70) ermöglicht, an dem die Verbindungen mit den Ambossarmen (52; 238) gekoppelt sind.

9. Vorrichtung nach Anspruch 6, wobei das Schneidsystem (14; 232) weiterhin Folgendes umfasst:
einen Riemenantrieb (80), der die Ambosswelle (76) antreibt, um eine Drehung des Ambossrades (46; 234) zu bewirken; und
einen Messerrollen-Servomotor (82), der mit der Messerrolle (48; 236) gekoppelt ist, um die Drehung der Messerrolle (48; 236) anzutreiben;
wobei der Messerwalzen-Servomotor (82) vom Riemenantrieb (80) getrennt ist, so dass die Messerrolle (48; 236) getrennt vom Ambossrad (46; 234) über die Nockensteuerung des Messerrollen-Servomotors (82) angetrieben wird,
und optional weiterhin Folgendes umfasst:
eine untere Schneidkastenanordnung (86) mit entlang ihrer Oberseite ausgebildeten Schienen (88);
eine obere Schneidkastenanordnung (84), die über der unteren Schneidkastenanordnung (86) positioniert und relativ zur unteren Schneidkastenanordnung (86) entlang der Schienen (88) seitlich beweglich ist; und
einen Einstellmechanismus, der betätigt werden kann, um eine seitliche Bewegung der oberen Schneidkastenanordnung (84) entlang der Schienen (88) zu bewirken;
wobei das Ambossrad (46; 234) und die Messerwalze (48; 236) jeweils an der oberen Schneidkastenanordnung (84) montiert sind, so dass eine seitliche Bewegung der oberen Schneidkastenanordnung (84) dazu führt, dass sich das Ambossrad (46; 234) und die Messerwalze (48; 236) relativ zum Übertragungsmechanismus seitlich bewegen.

10. Vorrichtung (10) nach Anspruch 6, weiterhin umfassend eine Mensch-Maschine-Schnittstelle (HMI) (18), die dazu konfiguriert ist:
Bedienereingaben zu empfangen, die auf eine Neukonfiguration des Übertragungsmechanismus (12) und/oder des Schneidsystems (14; 232) gerichtet sind; und
als Reaktion auf die Bedienereingaben Befehle zu erzeugen, die dazu führen, dass eine oder mehrere Betriebseinstellungen des Übertragungsmechanismus (12) und/oder des Schneidsystems (14; 232) geändert werden;
wobei die eine oder mehreren Betriebseinstellungen mindestens eines von einer Drehzahl der Antriebswelle (28), einem Abstand zwischen benachbarten Pucks (22) und einer Drehzahl der Vielzahl von Servomotoren (106), einer seitlichen Positionierung des Ambossrades (46; 234) und der Messerwalze (48; 236), einer Drehzahl der Ambosswelle (76) und einer Drehzahl des Messerwalzen-Servomotors (82) umfassen,
und optional weiterhin umfassend einen Schleifring (124), der auf der Antriebswelle (28) positioniert ist, und wobei Kommunikations- und/oder Stromverbindungen zu der Vielzahl von Servomotoren (106) durch den Schleifring (124) geführt werden.

11. Vorrichtung (10) nach Anspruch 1, wobei der Verbindungsarm (114) einen gekrümmten, bogenförmigen Verbindungsarm umfasst.

12. Bestückungssystem (250) zum Übertragen und Drehen einer Vielzahl einzelner Artikel von mindestens einem Artikelaufnahmeort zu einer Artikelplatzierungsstelle, wobei das Bestückungssystem Folgendes umfasst:
eine Antriebswelle (28), die um eine Übertragungsachse (32) drehbar ist;
eine Mittelplatte (24), die an der Antriebswelle (28) montiert ist, um sich mit dieser um die Übertragungsachse (32) zu drehen;
eine Vielzahl von Wageneinheiten (20), die um die Mittelplatte (24) positioniert sind, um sich mit dieser zu drehen und entlang eines Übertragungswegs um die Übertragungsachse (32) von mindestens der Artikelaufnahmestelle zur Artikelplatzierungsstelle zu fahren, wobei jede der Vielzahl von Wageneinheiten (20) einen Puck (22) enthält, der selektiv betätigt werden kann, um einen einzelnen Artikel darauf zu tragen und den Artikel neu auszurichten, während sich der Puck (22) zwischen der Artikelaufnahmestelle und der Artikelplatzierungsstelle bewegt; und
eine Vielzahl von Servomotoren (106), die an der Mittelplatte (24) montiert sind, wobei jeder Servomotor über einen Verbindungsarm (114) betriebsmäßig mit einer jeweiligen Wageneinheit der Vielzahl von Wageneinheiten (20) verbunden ist, und wobei jeder der Vielzahl von Servomotoren (106) betreibbar ist, um die Positionierung seiner jeweiligen Wageneinheit in Bezug auf die Mittelplatte (24) entlang zumindest eines Abschnitts des Übertragungswegs zu ändern.

13. Bestückungssystem (250) nach Anspruch 12, wobei die Mittelplatte (24) eine ringförmige Schienenstruktur umfasst, die um ihren Außenumfang herum positioniert ist, wobei die Schienenstruktur Neigungsschienen (96) umfasst, auf denen die Vielzahl von Wageneinheiten (20) so montiert ist, dass sie entlang dieser beweglich sind.

14. Bestückungssystem (250) nach Anspruch 13, wobei jede der Vielzahl von Wageneinheiten (20) Folgendes umfasst:
ein Fahrgestell mit einem Paar Rahmenelementen, wobei die Rahmenelemente auf gegenüberliegenden Seiten der Schienenstruktur positioniert sind;
eine Vielzahl von Rollen, die mit dem Fahrgestell verbunden sind und in Rolleingriff mit einer Innenkante und einer Außenkante der Neigungsschienen (96) stehen, um die Wageneinheit an der Schienenkonstruktion zu befestigen und um eine Verschiebung der Wageneinheit entlang der Neigungsschienen (96) zu ermöglichen; und
eine Puckhalterung, die auf dem Fahrgestell positioniert und relativ zu diesem über eine Verbindung von Schienenführungen der Puckhalterung mit den Neigungsschienen (96) beweglich ist, wobei die Puckhalterung im Allgemeinen orthogonal zu den Neigungsschienen (96) ausgerichtet ist und an einem Ende davon eine Puckhalterung umfasst, die zur Aufnahme des Pucks (22) konfiguriert ist.

15. Bestückungssystem nach Anspruch 13, umfassend:
einen stationären Vakuumverteiler, der neben der Mittelplatte (24) positioniert und so konfiguriert ist, dass er ein Vakuum von einer Vakuumquelle in ein Innenvolumen der Mittelplatte (24) überträgt; und
eine Vielzahl von Vakuumröhren, die mit der Mittelplatte (24) gekoppelt sind, wobei eine oder mehrere Vakuumröhren der Vielzahl von Vakuumröhren das Innenvolumen der Mittelplatte (24) mit einer entsprechenden Wageneinheit der Vielzahl von Wageneinheiten (20) strömungstechnisch verbinden, um das Vakuum darauf zu übertragen;
wobei jede Vakuumröhre drehbar mit der Mittelplatte (24) gekoppelt ist, um relativ dazu zu schwenken und wobei eine Länge und/oder Konfiguration jeder Vakuumröhre variabel ist, um der Bewegung einer jeweiligen Wageneinheit entlang der Schienenstruktur Rechnung zu tragen.

16. Bestückungssystem nach Anspruch 13, weiterhin umfassend:
eine Vielzahl von Motorantrieben, die an der Mittelplatte (24) befestigt und betriebsmäßig mit den Vielzahl von Servomotoren (106) verbunden ist, um deren Betrieb zu steuern; und
eine Mensch-Maschine-Schnittstelle (HMI), die mit den Vielzahl von Motorantrieben kommuniziert, um ihnen Befehle zu übermitteln, wobei die HMI dazu konfiguriert ist:
Bedienereingaben zu empfangen, die auf eine Neukonfiguration des Bestückungssystem gerichtet sind; und
der Vielzahl von Motorantrieben Befehle zu geben, um die Vielzahl von Servomotoren (106) gemäß einem vorbestimmten Geschwindigkeitsprofil zu steuern, das den Bedienereingaben zugeordnet ist, und dadurch die Positionierung der Vielzahl von Wageneinheiten (20) in Bezug auf die Mittelplatte (24) entlang zumindest des Abschnitts des Übertragungswegs zu ändern.

17. Verfahren zum Konfigurieren einer Schneid- und Übertragungsvorrichtung (10), wobei das Verfahren Folgendes umfasst:
Bereitstellen eines Schneidmechanismus (14), der so konfiguriert ist, dass er eine ankommende Materialbahn in eine Vielzahl einzelner Artikel schneidet; und
Bereitstellen eines Übertragungsmechanismus (12), der mit dem Schneidmechanismus (14) betreibbar ist, um die Vielzahl einzelner Artikel von mindestens einer Bahnaufnahmestelle zu einer Artikelplatzierungsstelle zu übertragen und zu drehen, wobei das Bereitstellen des Übertragungsmechanismus (12) Folgendes umfasst:
Bereitstellen einer Antriebswelle (28) mit einer daran gekoppelten Montagestruktur, wobei die Antriebswelle (28) und die Montagestruktur um eine Übertragungsachse (32) drehbar sind; und
Montieren einer Vielzahl von Wageneinheiten (20) an der Montagestruktur, so dass die Vielzahl von Wageneinheiten (20) mit der Montagestruktur drehbar sind, um sich entlang eines Übertragungswegs (34) um die Übertragungsachse (32) von mindestens der Bahnaufnahmestelle zur Kissenplatzierungsstelle zu bewegen, wobei jede der Vielzahl von Wageneinheiten (20) einen Puck (22) umfasst, der selektiv betätigt werden kann, um einen einzelnen Artikel darauf zu tragen und den Artikel neu auszurichten, während sich der Puck (22) zwischen der Bahnaufnahmestelle und der Artikelplatzierungsstelle bewegt; und
betriebsfähiges Verbinden eines Servomotors (106) mit jeder der Vielzahl von Wageneinheiten (22) über einen Verbindungsarm (114), wobei jeder Servomotor selektiv betreibbar ist, um die Positionierung seiner jeweiligen Wageneinheit in Bezug auf die Montagestruktur zu ändern, indem er die Wageneinheit entlang des Übertragungswegs (34) in Umfangsrichtung verschiebt.

18. Verfahren nach Anspruch 17, wobei die Montagestruktur eine Mittelplatte (24) umfasst und wobei das Montieren der Vielzahl von Wageneinheiten (20) das Montieren der Vielzahl von Wageneinheiten (20) auf einer ringförmigen Schienenstruktur (94) umfasst, die um einen Außenumfang der Mittelplatte (24) herum positioniert ist, wobei die Schienenstruktur (94) Neigungsschienen (96) umfasst, auf denen die Vielzahl von Wageneinheiten (20) montiert sind, um entlang dieser beweglich zu sein.

19. Verfahren nach Anspruch 17, weiterhin umfassend Ankoppeln eines jeweiligen Pucks (22) an jede der Vielzahl von Wageneinheiten (20), um Pucks (22) bereitzustellen, die eine gewünschte Artikelgröße aufnehmen, die von der Schneid- und Übertragungsvorrichtung (10) hergestellt werden soll.

20. Verfahren nach Anspruch 18, weiterhin umfassend:
Bereitstellen von Eingaben für die Schneide- und Übertragungsvorrichtung (10) von einer Mensch-Maschine-Schnittstelle (HMI) (18), die eine Produktgröße angeben, die von der Schneide- und Übertragungsvorrichtung (10) hergestellt werden soll; und
Positionieren des Schneidmechanismus (14) relativ zum Übertragungsmechanismus (12) basierend auf den Eingaben von der HMI (18), wobei das Positionieren des Schneidmechanismus (14) das Positionieren eines Ambossrads (46; 234) und einer Messerrolle (48; 236) des Schneidmechanismus (14) relativ zum Übertragungsmechanismus (12) basierend auf den Eingaben von der HMI (18) umfasst, um die ankommende Materialbahn an einer Schneidstelle zu schneiden.
und optional weiter umfassend:
Steuern einer Drehzahl der Antriebswelle (28) und des Ambossrades (46; 234) basierend auf den Eingaben von der HMI (18), wobei die Drehzahl der Antriebswelle (28) und die Drehzahl des Ambossrades (46; 234) gleich gehalten werden;
Steuern einer Drehzahl der Servomotoren (106) basierend auf den Eingaben von der HMI (18); und
Steuern einer Drehgeschwindigkeit der Messerrolle (48; 236) über die Nockensteuerung eines Messerrollen-Servomotors (82), der operativ mit der Messerrolle (48; 236) verbunden ist, basierend auf den Eingaben vom HMI (18) .

21. Verfahren zur Einleitung eines Produktwechsels in einer Übertragungsvorrichtung (10), wobei die Übertragungsvorrichtung (10) ein Puckrad (26) und ein Ambossrad (46; 234) umfasst, die von mindestens einer Antriebswelle (28) angetrieben werden, wobei eine Vielzahl von Wageneinheiten (20) entlang eines Umfangs des Puckrads (26) betriebsmäßig gekoppelt ist, wobei jede Wageneinheit einen austauschbaren Puck (22) enthält, wobei das Ambossrad (46; 234) eine Vielzahl von Ambossarmen (52; 238), die schwenkbar mit einer Ambossnabe (50; 242) verbunden sind und sich von der Ambossnabe (50; 242) radial nach außen erstrecken, und eine rotierende Klinge im Zusammenwirken mit den Ambossarmen (52; 238) aufweist, wobei das Verfahren Folgendes umfasst:
optionales Entfernen jedes Pucks (22) mit einer ersten Größe von seiner entsprechenden Wageneinheit;
optional Anbringen eines Pucks (22) mit einer zweiten Größe an jeder entsprechenden Wageneinheit;
Senden von Befehlen nacheinander oder gleichzeitig an eine oder mehrere Steuerungen, die einen Antriebswellenmotor, eine Vielzahl von Motoren der Wageneinheit und einen Klingenmotor steuern, um:
eine Drehzahl der Antriebswelle (26) zu modifizieren, um die Geschwindigkeit des Puckrads (26) und des Ambossrads (46; 234) zu modifizieren;
eine ausgewählte Wageneinheit zu beschleunigen, um die ausgewählte Wageneinheit linear von einer ersten Position in eine zweite Position entlang eines Teils des Umfangs des Puckrads (26) zu verschieben, wobei die Verschiebung der ausgewählten Wageneinheit eine Winkelposition der entsprechenden Ambossarme (52; 238) neben der ausgewählten Wageneinheit ändert;
eine Drehzahl und Winkelposition des rotierenden Messers einzustellen, so dass die rotierende Klinge auf ein distales Ende eines entsprechenden Ambossarms (52; 238) trifft und eine kontinuierliche Materialbahn schneidet, die zwischen der rotierenden Klinge und dem distalen Ende des entsprechenden Ambossarms (52; 238) angeordnet ist; und
eine ausgewählte Wageneinheit zu entschleunigen, um die ausgewählte Wageneinheit linear von der zweiten Position in die erste Position zu verschieben.

22. Verfahren nach Anspruch 21, wobei der Schritt des optionalen Entfernens jedes Pucks (22) der ersten Größe und des Anbringens jedes Pucks (22) der zweiten Größe durch einen Robotermechanismus oder durch einen Menschen durchgeführt wird.

23. Verfahren nach Anspruch 21, das das Versehen jeder Wageneinheit mit einer Schnellverbindungs-Eingriffsstruktur oder einer befestigungslosen Befestigung einschließt, um ein effizientes Auswechseln jedes Pucks (22) auf seiner entsprechenden Wageneinheit zu ermöglichen.

24. Verfahren nach Anspruch 21, weiterhin umfassend das Bereitstellen eines Servomotors (106), um die Beschleunigung jeder Wageneinheit zu steuern.

25. Verfahren nach Anspruch 21, weiterhin umfassend Bereitstellen eines Servomotors (82), der eine Drehzahl und Winkelposition des rotierenden Messers steuert, so dass die rotierende Klinge auf ein distales Ende eines entsprechenden Ambossarms (52; 238) trifft und die kontinuierliche Materialbahn schneidet, die zwischen der rotierenden Klinge und dem distalen Ende des entsprechenden Ambossarms (52; 238) angeordnet ist.

## Revendications

1. Appareil de coupe et de transfert (10 ; 230) comprenant :
un système de coupe (14 ; 232) configuré pour couper une bande entrante continue de matériau en une pluralité d'articles discrets ; et
un mécanisme de transfert (12) pouvant fonctionner avec le système de coupe (14 ; 232) pour transférer et faire tourner la pluralité d'articles discrets depuis au moins un emplacement de réception de bande vers un emplacement de placement d'article, le mécanisme de transfert (12) comprenant :
un arbre d'entraînement (28) pouvant tourner autour d'un axe de transfert (32) ;
une roue à palet (26) montée sur l'arbre d'entraînement (28) pour tourner avec celui-ci autour de l'axe de transfert (32), la roue à palet (26) comportant :
une pluralité d'unités de chariot (20) qui tournent autour de l'axe de transfert (32) pour se déplacer le long d'un chemin de transfert (34) autour de l'axe de transfert (32) depuis au moins l'emplacement de réception de bande jusqu'à l'emplacement de placement d'article, chacune de la pluralité d'unités de chariot (20) comportant un palet (22) qui peut fonctionner de manière sélective pour transporter un article discret sur celle-ci et réorienter l'article lorsque le palet (22) se déplace entre l'emplacement de réception de bande et l'emplacement de placement d'article ; et
une pluralité de servomoteurs (106), chacun étant connecté fonctionnellement à une unité de chariot respective de la pluralité d'unités de chariot (20) via un bras de connexion (114), et avec chacun de la pluralité de servomoteurs (106) pouvant fonctionner pour modifier le positionnement de son unité de chariot respective le long d'au moins une partie du chemin de transfert (34).

2. Appareil (10) selon la revendication 1, dans lequel la roue à palet (26) comprend une plaque centrale (24) montée sur l'arbre d'entraînement (28) pour tourner avec celui-ci autour de l'axe de transfert (32), avec la pluralité d'unités de chariot (20) montée sur et positionnée autour de la plaque centrale (24) et avec la pluralité de servomoteurs (106) montée sur la plaque centrale (24) ; et dans lequel chacun de la pluralité de servomoteurs (106) peut fonctionner pour modifier le positionnement de son unité de chariot respective par rapport à la plaque centrale (24) le long d'au moins la partie du chemin de transfert (34).

3. Appareil (10) selon la revendication 2, comprenant en outre une structure de rail annulaire (94) positionnée autour d'une circonférence externe de la plaque centrale (24), la structure de rail (94) comportant des maillons de chenille (96) sur lesquels la pluralité d'unités de chariot (20) est montée mobile le long de ceux-ci.

4. Appareil (10) selon la revendication 3, dans lequel chacune de la pluralité d'unités de chariot (20) comprend :
un châssis (132) comportant une paire d'éléments de cadre (136, 138) positionnés sur des côtés opposés de la structure de rail (94) ;
une pluralité de rouleaux (140, 142) couplés au châssis (132) et en prise de roulement avec un bord interne (100) et un bord externe (102) des maillons de chenille (96) pour fixer l'unité de chariot à la structure de rail (94) et assurer la translation de l'unité de chariot le long des maillons de chenille (96) ; et
un support de palet (134) positionné sur le châssis (132) et mobile par rapport à celui-ci via un accouplement de rails de guidage du support de palet (134) avec les maillons de chenille (96), le support de palet (134) étant orienté généralement orthogonalement aux maillons de chenille (96) et comprenant un support de palet (144) sur une extrémité de celui-ci qui est configuré pour recevoir le palet (22).

5. Appareil (10) selon la revendication 4, dans lequel le mécanisme de transfert (12) comprend :
un collecteur de vide stationnaire (42) positionné adjacent à la plaque centrale (24) et configuré pour transférer un vide d'une source de vide vers un volume intérieur de la plaque centrale (24) ; et
une pluralité de tubes à vide couplés à la plaque centrale (24), avec un ou plusieurs tubes à vide de la pluralité de tubes à vide connectant fluidiquement le volume intérieur de la plaque centrale (24) à une unité de chariot respective de la pluralité d'unités de chariot (20) pour transférer le vide à celle-ci,
dans lequel, éventuellement, la pluralité de tubes à vide comprennent des tubes à vide télescopiques (44), chacun étant couplé de manière rotative à la plaque centrale (24) pour pivoter par rapport à celle-ci, et dans lequel chaque tube à vide télescopique (44) est pliable et extensible pour diminuer et augmenter une longueur de celui-ci, de sorte qu'une longueur du tube à vide télescopique (44) peut changer pour s'adapter au mouvement d'une unité de chariot respective le long de la structure de rail (94).

6. Appareil (10 ; 230) selon la revendication 1, dans lequel le système de coupe (14 ; 232) comprend :
un rouleau à couteaux (48 ; 236) comportant un ou plusieurs couteaux (58) sur celui-ci ; et
une roue enclume (46 ; 234), comprenant :
un moyeu d'enclume (50 ; 242) couplé à et entraîné par un arbre d'enclume (76) pour tourner autour d'un axe de roue enclume (81 ; 244) ;
une pluralité de bras d'enclume (52 ; 238) connectés de manière pivotante au moyeu d'enclume (50 ; 242) et s'étendant radialement vers l'extérieur à partir de celui-ci ; et
une enclume (56 ; 240) positionnée au niveau d'une extrémité de chacun de la pluralité de bras d'enclume (52 ; 238), les enclumes (56) coopérant périodiquement avec les un ou plusieurs couteaux (58) du rouleau à couteaux (48 ; 236) pour couper la bande entrante de matériau ; et
un système de liaison (64) qui couple mécaniquement la pluralité de bras d'enclume (52 ; 238) ensemble.

7. Appareil (10) selon la revendication 6, dans lequel le système de liaison (64) comprend une paire de liaisons en ciseaux (66) prévues entre chaque paire adjacente de bras d'enclume de la pluralité de bras d'enclume (52 ; 238), avec une première liaison de la paire de liaisons en ciseaux (66) couplée de manière rotative à un premier bras d'enclume de la paire adjacente de bras d'enclume (52 ; 238) et une seconde liaison de la paire de liaisons en ciseaux (66) couplée de manière rotative à un second bras d'enclume de la paire adjacente de bras d'enclume (52 ; 238).

8. Appareil (10) selon la revendication 7, dans lequel chacun de la pluralité de bras d'enclume (52 ; 238) comprend un coulisseau linéaire (71) par lequel des liaisons respectives du système de liaison (64) sont couplées à celui-ci, le coulisseau linéaire (71) fournissant un mouvement radialement vers l'intérieur et vers l'extérieur d'un point de pivotement (70) auquel les liaisons sont couplées aux bras d'enclume (52 ; 238).

9. Appareil selon la revendication 6, dans lequel le système de coupe (14 ; 232) comprend en outre :
un entraînement par courroie (80) qui entraîne l'arbre d'enclume (76) pour entraîner la rotation de la roue enclume (46 ; 234) ; et
un servomoteur de rouleau à couteaux (82) couplé au rouleau à couteaux (48 ; 236) pour entraîner la rotation du rouleau à couteaux (48 ; 236) ;
dans lequel le servomoteur de rouleau à couteaux (82) est séparé de l'entraînement par courroie (80), de sorte que le rouleau à couteaux (48 ; 236) est entraîné séparément de la roue enclume (46 ; 234) via un déplacement par came du servomoteur de rouleau à couteaux (82),
et, éventuellement, comprend en outre :
un ensemble boîte de coupe inférieure (86) ayant des pistes (88) formées le long d'une surface supérieure de celui-ci ;
un ensemble boîte de coupe supérieure (84) positionné au-dessus de l'ensemble boîte de coupe inférieure (86) et mobile latéralement par rapport à l'ensemble boîte de coupe inférieure (86) le long des pistes (88) ; et
un mécanisme de réglage pouvant être actionné pour entraîner un mouvement latéral de l'ensemble boîte de coupe supérieure (84) le long des pistes (88) ;
dans lequel chacun de la roue enclume (46 ; 234) et du rouleau à couteaux (48 ; 236) est monté sur l'ensemble boîte de coupe supérieure (84), de sorte qu'un mouvement latéral de l'ensemble boîte de coupe supérieure (84) amène la roue enclume (46 ; 234) et le rouleau à couteaux (48 ; 236) à se déplacer latéralement par rapport au mécanisme de transfert.

10. Appareil (10) selon la revendication 6, comprenant en outre une interface homme-machine (IHM) (18) configurée pour :
recevoir des entrées d'opérateur dirigées vers une reconfiguration du mécanisme de transfert (12) et/ou du système de coupe (14 ; 232) ; et
générer des commandes en réponse aux entrées d'opérateur qui entraînent la modification d'un ou de plusieurs réglages opérationnels du mécanisme de transfert (12) et/ou du système de coupe (14 ; 232) ;
dans lequel les un ou plusieurs réglages opérationnels comprennent au moins l'un parmi une vitesse de rotation de l'arbre d'entraînement (28), un pas entre des palets adjacents (22), une vitesse de la pluralité de servomoteurs (106), un positionnement latéral de la roue enclume (46 ; 234) et du rouleau à couteaux (48 ; 236), une vitesse de rotation de l'arbre d'enclume (76) et une vitesse du servomoteur de rouleau à couteaux (82),
et, éventuellement, comprenant en outre une bague collectrice (124) positionnée sur l'arbre d'entraînement (28), et dans lequel des liaisons de communications et/ou d'alimentation vers la pluralité de servomoteurs (106) sont acheminées à travers la bague collectrice (124).

11. Appareil (10) selon la revendication 1, dans lequel le bras de connexion (114) comprend un bras de connexion incurvé et de forme arquée.

12. Système de prélèvement et de placement (250) pour transférer et faire tourner une pluralité d'articles discrets depuis au moins un emplacement de réception d'article vers un emplacement de placement d'article, le système de prélèvement et de placement comprenant :
un arbre d'entraînement (28) pouvant tourner autour d'un axe de transfert (32) ;
une plaque centrale (24) montée sur l'arbre d'entraînement (28) pour tourner avec celui-ci autour de l'axe de transfert (32) ;
une pluralité d'unités de chariot (20) positionnées autour de la plaque centrale (24) pour tourner avec celle-ci pour se déplacer le long d'un chemin de transfert autour de l'axe de transfert (32) depuis au moins l'emplacement de réception d'article jusqu'à l'emplacement de placement d'article, chacun de la pluralité d'unités de chariot (20) comprenant un palet (22) qui peut être actionnée de manière sélective pour transporter un article discret sur celle-ci et réorienter l'article lorsque le palet (22) se déplace entre l'emplacement de réception d'article et l'emplacement de placement d'article ; et
une pluralité de servomoteurs (106) montés sur la plaque centrale (24), chaque servomoteur étant connecté de manière fonctionnelle à une unité de chariot respective de la pluralité d'unités de chariot (20) via un bras de connexion (114), et avec chacun de la pluralité de servomoteurs (106) pouvant fonctionner pour modifier le positionnement de son unité de chariot respective par rapport à la plaque centrale (24) le long d'au moins une partie du chemin de transfert.

13. Système de prélèvement et de placement (250) selon la revendication 12, dans lequel la plaque centrale (24) comprend une structure de rail annulaire positionnée autour d'une circonférence externe de celle-ci, la structure de rail comportant des maillons de chenille (96) sur lesquels la pluralité d'unités de chariot (20) est montée pour être mobile le long de ceux-ci.

14. Système de prélèvement et de placement (250) selon la revendication 13, dans lequel chacune de la pluralité d'unités de chariot (20) comprend :
un châssis comportant une paire d'éléments de cadre, les éléments de cadre étant positionnés sur des côtés opposés de la structure de rail ;
une pluralité de rouleaux couplés au châssis et en prise de roulement avec un bord interne et un bord externe des maillons de chenille (96) pour fixer l'unité de chariot à la structure de rail et assurer la translation de l'unité de chariot le long des maillons de chenille (96) ; et
un support de palet positionné sur le châssis et mobile par rapport à celui-ci via un accouplement de rails de guidage du support de palet avec les maillons de chenille (96), le support de palet étant orienté généralement orthogonalement aux maillons de chenille (96) et comprenant un support de palet sur une extrémité celui-ci qui est configuré pour recevoir le palet (22).

15. Système de prélèvement et de placement selon la revendication 13, comprenant :
un collecteur de vide stationnaire positionné adjacent à la plaque centrale (24) et configuré pour transférer un vide d'une source de vide vers un volume intérieur de la plaque centrale (24) ; et
une pluralité de tubes à vide couplés à la plaque centrale (24), avec un ou plusieurs tubes à vide de la pluralité de tubes à vide connectant fluidiquement le volume intérieur de la plaque centrale (24) à une unité de chariot respective de la pluralité d'unités de chariot (20) pour transférer le vide à celle-ci ;
dans lequel chaque tube à vide est couplé de manière rotative à la plaque centrale (24) pour pivoter par rapport à celle-ci, et dans lequel une longueur et/ou une configuration de chaque tube à vide est variable pour s'adapter au mouvement d'une unité de chariot respective le long de la structure de rail.

16. Système de prélèvement et de placement selon la revendication 13 comprenant en outre :
une pluralité d'entraînements de moteur fixés sur la plaque centrale (24) et connectés de manière fonctionnelle à la pluralité de servomoteurs (106) pour commander le fonctionnement de ceux-ci ; et
une interface homme-machine (IHM) en communication avec la pluralité d'entraînements de moteur pour leur fournir des commandes, l'IHM étant configurée pour :
recevoir des entrées d'opérateur dirigées vers une reconfiguration du système de prélèvement et de placement ; et
fournir des commandes à la pluralité d'entraînements de moteur pour réaliser un déplacement par came de la pluralité de servomoteurs (106) selon un profil de vitesse prédéterminé associé aux entrées d'opérateur et modifier ainsi le positionnement de la pluralité d'unités de chariot (20) par rapport à la plaque centrale (24) le long d'au moins une partie du chemin de transfert.

17. Procédé de configuration d'un appareil de coupe et de transfert (10), le procédé comprenant :
la fourniture d'un mécanisme de coupe (14) configuré pour couper une bande entrante de matériau en une pluralité d'articles discrets ; et
la fourniture d'un mécanisme de transfert (12) pouvant fonctionner avec le mécanisme de coupe (14) pour transférer et faire tourner la pluralité d'articles discrets depuis au moins un emplacement de réception de bande vers un emplacement de placement d'article, la fourniture du mécanisme de transfert (12) comprenant :
la fourniture d'un arbre d'entraînement (28) auquel est couplée une structure de montage, l'arbre d'entraînement (28) et la structure de montage pouvant tourner autour d'un axe de transfert (32) ; et
le montage d'une pluralité d'unités de chariot (20) sur la structure de montage de sorte que la pluralité d'unités de chariot (20) peuvent tourner avec la structure de montage pour se déplacer le long d'un chemin de transfert (34) autour de l'axe de transfert (32) à partir au moins de l'emplacement de réception de bande vers l'emplacement de placement de tampon, chacune de la pluralité d'unités de chariot (20) comportant un palet (22) qui peut fonctionner de manière sélective pour transporter un article discret sur celle-ci et réorienter l'article lorsque le palet (22) se déplace entre l'emplacement de réception de bande et l'emplacement de placement d'article ; et
la connexion de manière fonctionnelle d'un servomoteur (106) à chacune de la pluralité d'unités de chariot (22) via un bras de connexion (114), chaque servomoteur pouvant fonctionner de manière sélective pour modifier le positionnement de son unité de chariot respective par rapport à la structure de montage par déplacement circonférentiel de l'unité de chariot le long du chemin de transfert (34).

18. Procédé selon la revendication 17, dans lequel la structure de montage comprend une plaque centrale (24) et dans lequel le montage de la pluralité d'unités de chariot (20) comprend le montage de la pluralité d'unités de chariot (20) sur une structure de rail annulaire (94) positionnée autour d'une circonférence externe de la plaque centrale (24), la structure de rail (94) comportant des maillons de chenille (96) sur lesquels la pluralité d'unités de chariot (20) est montée pour être mobile le long de ceux-ci.

19. Procédé selon la revendication 17, comprenant en outre le couplage d'un palet respectif (22) à chacune de la pluralité d'unités de chariot (20) pour fournir des palets (22) qui s'adaptent à une taille d'article souhaitée qui doit être produite par l'appareil de coupe et de transfert (10).

20. Procédé selon la revendication 18, comprenant en outre :
la fourniture d'entrées à l'appareil de coupe et de transfert (10) à partir d'une interface homme-machine (IHM) (18) indiquant une taille de produit à produire par l'appareil de coupe et de transfert (10) ; et
le positionnement du mécanisme de coupe (14) par rapport au mécanisme de transfert (12) sur la base des entrées provenant de l'IHM (18), dans lequel le positionnement du mécanisme de coupe (14) comprend le positionnement d'une roue enclume (46 ; 234) et d'un rouleau à couteaux (48 ; 236) du mécanisme de coupe (14) par rapport au mécanisme de transfert (12) sur la base des entrées provenant de l'IHM (18) pour couper la bande entrante de matériau au niveau d'un emplacement de coupe.
et, éventuellement, comprenant en outre :
la commande d'une vitesse de rotation de l'arbre d'entraînement (28) et de la roue enclume (46 ; 234) sur la base des entrées provenant de l'IHM (18), de la vitesse de rotation de l'arbre d'entraînement (28) et de la vitesse de rotation de la roue enclume (46 ; 234) étant maintenues égales ;
la commande d'une vitesse de rotation des servomoteurs (106) sur la base des entrées provenant de l'IHM (18) ; et
la commande d'une vitesse de rotation du rouleau à couteaux (48 ; 236), via un déplacement par came d'un servomoteur de rouleau à couteaux (82) connecté fonctionnellement au rouleau à couteaux (48 ; 236), sur la base des entrées provenant de l'IHM (18).

21. Procédé pour initier un changement de produit dans un appareil de transfert (10), l'appareil de transfert (10) comportant une roue à palet (26) et une roue enclume (46 ; 234) entraînée par au moins un arbre d'entraînement (28), une pluralité d'unités de chariot (20) couplées fonctionnellement le long d'une circonférence de la roue à palet (26), chaque unité de chariot comportant un palet interchangeable (22), la roue enclume (46 ; 234) ayant une pluralité de bras d'enclume (52 ; 238) connectés de manière pivotante à un moyeu d'enclume (50 ; 242) et s'étendant radialement vers l'extérieur à partir du moyeu d'enclume (50 ; 242), et une lame rotative en coopération avec les bras d'enclume (52 ; 238), le procédé comprenant :
le retrait éventuel de chaque palet (22) ayant une première taille de son unité de chariot correspondante ;
la fixation éventuelle d'un palet (22) ayant une seconde taille à chaque unité de chariot correspondante ;
l'envoi de commandes, séquentiellement ou simultanément, à un ou plusieurs dispositifs de commande qui commandent un moteur d'arbre d'entraînement, une pluralité de moteurs d'unité de chariot et un moteur de lame, pour :
modifier une vitesse de rotation de l'arbre d'entraînement (26) pour modifier la vitesse de la roue à palet (26) et de la roue enclume (46 ; 234) ;
accélérer une unité de chariot sélectionnée pour déplacer linéairement l'unité de chariot sélectionnée d'une première position à une seconde position le long d'une partie de la circonférence de la roue à palet (26), dans lequel le déplacement de l'unité de chariot sélectionnée modifie une position angulaire de bras d'enclume correspondants (52 ; 238) adjacents à l'unité de chariot sélectionnée ;
régler une vitesse de rotation et une position angulaire de la lame rotative de sorte que la lame rotative rencontre une extrémité distale d'un bras d'enclume correspondant (52 ; 238), et coupe une bande continue de matériau disposée entre la lame rotative et l'extrémité distale du bras d'enclume correspondant (52 ; 238) ; et
décélérer une unité de chariot sélectionnée pour déplacer linéairement l'unité de chariot sélectionnée de la seconde position à la première position.

22. Procédé selon la revendication 21, dans lequel l'étape de retrait éventuel de chaque palet (22) de la première taille et de fixation éventuelle de chaque palet (22) de la seconde taille est réalisée par un mécanisme robotique ou par un être humain.

23. Procédé selon la revendication 21, comportant la fourniture à chaque unité de chariot d'une structure de mise en prise de connecteur rapide ou d'une attache sans fixation pour faciliter un échange efficace de chaque palet (22) sur son unité de chariot correspondante.

24. Procédé selon la revendication 21, comprenant en outre la fourniture d'un servomoteur (106) pour commander l'accélération de chaque unité de chariot.

25. Procédé selon la revendication 21, comprenant en outre la fourniture d'un servomoteur (82) commander une vitesse de rotation et une position angulaire de la lame rotative de sorte que la lame rotative rencontre une extrémité distale d'un bras d'enclume correspondant (52 ; 238), et coupe la bande de matériau continue disposée entre la lame rotative et l'extrémité distale du bras d'enclume correspondant (52 ; 238).
